# FASCICULE DE BREVET EUROPEEN

(11) **EP 1 468 670 B1**
(45) Date de publication et mention de la délivrance du brevet: **24.01.2007**
(21) Numéro de dépôt: 04290647.9
(22) Date de dépôt: 10.03.2004
(51) Int. Cl.: A61K 8/37, A61K 8/81, A61Q 17/04

(54) **Composition photoprotectrice comprenant au moins un polymère d'acide acrylamido 2-méthyl propane sulfonique et un 4,4-diarylbutadiène, Utilisations**
A sunscreen composition comprising at least a acrylamido 2-methylpropane sulphonic acid polymer and 4,4-diarylbutadien, and use thereof
Ein Lichtschutzmittel, welches mindestens ein Akrylamido-2-methylpropan sulfonsäure Polymer und 4,4-Diarylbutadien enthält, und die Verwendung davon

(30) Priorité: 14.04.2003 FR 0304647
(43) Date de publication de la demande: 20.10.2004
(73) Titulaire: L'ORÉAL, 75008 Paris (FR)
(72) Inventeur: L'Alloret, Florence, 75013 Paris (FR)
(74) Mandataire: Miszputen, Laurent

(56) Documents cités:
- DE-A- 10 007 017
- DE-A- 10 124 914
- DE-A- 19 820 116
- US-A1- 2003 031 643
- US-B1- 6 436 373

## Description

L'invention concerne une composition photoprotectrice sous forme d'émulsion huile-dans-eau comprenant au moins une phase aqueuse, au moins une phase huileuse, au moins un polymère hydrosoluble ou hydrodispersible d'acide acrylamido 2-méthyl propane sufonique, partiellement ou totalement neutralisé, réticulé ou non-réticulé et au moins un système filtrant les radiations UV, caractérisée par le fait que le système filtrant comprend au moins un filtre UV-A du type 4,4-diarylbutadiène.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques liposolubles et/ou des filtres organiques classiques hydrosolubles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

Les polymères de l'acide acrylamido 2-méthyl propane sulfonique (AMPS) sont particulièrement appréciés pour leurs propriétés cosmétiques et leur flexibilité en terme de formulation par rapport aux dérivés polymériques de l'acide carboxylique (Carbopols fournis par Novéon). Ils peuvent en effet être formulés à des pH compris entre 4 et 6 sans que leurs propriétés gélifiantes soient diminuées. Parmi ces dérivés, on peut citer deux grande familles :
(a) les polymères hydrophiles ou hydrodispersibles tels que l'acide polyacrylamido 2-méthylpropane sulfonique neutralisé et réticulé, fourni par Clariant sous la dénomination Hostacerin AMPS, ou les copolymères d'AMPS et d'autre(s) monomère(s) hydrophiles tel que le Sépigel 305 fourni par la société Seppic et l'Aristoflex AVC fourni par la société Clariant.
(b) les polymères amphiphiles comprenant de l'AMPS et des unités alkyles tel que ceux décrits dans la demande EP 1 069 142.

Ces polymères de l'acide acrylamido 2-méthyl propane sulfonique sont particulièrement adaptées dans la fabrication des émulsions. Ils permettent d'obtenir des formulations stables dans une large gamme de texture et de consistance allant des laits aux crèmes épaissies.

Parmi les filtres UV-A organiques disponibles, une famille de composés particulièrement efficaces dans l'UV-A est l'acide benzène 1,4-di(3-méthylidène-10-camphosulfonique) et ses différents sels, décrite notamment dans les demandes de brevets FR-A-2528420 et FR-A-2639347, ils sont capables en effet d'absorber les rayons ultraviolets de longueur d'ondes comprises entre 280 et 400 nm, avec des maxima d'absorption compris entre 320 et 400 nm, en particulier aux alentours de 345 nm.

Cependant, l'introduction de ce type de filtre UVA dans des émulsions photoprotectrices stabilisées et/ou épaissies par un polymère de l'acide acrylamido 2-méthyl propane sulfonique, peut conduire à une diminution significative de leur viscosité ou bien à leur déstabilisation.

Il apparaît ainsi nécessaire de disposer d'émulsions à base de polymères de l'acide acrylamido 2-méthyl propane sulfonique qui soient stables et qui puissent contenir des filtres organiques actifs dans l'UV-A d'efficacité comparable à celle de l'acide benzène 1,4-di(3-méthylidène-10-camphosulfonique) et ses différents sels sans les inconvénients énumérés ci-dessus.

Par « stable », on entend que les aspects macroscopique et microscopique de la composition ne sont pas modifiés après 1 mois à la température ambiante.

La Demanderesse a découvert de façon surprenante que des émulsions comprenant au moins un polymère d'acide acrylamido 2-méthyl propane sufonique, partiellement ou totalement neutralisé, réticulé ou non-réticulé et au moins un filtre UV-A du type 4,4-diarylbutadiène, répondent à ce besoin.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Cette découverte est à la base de la présente invention.

Ainsi, conformément à l'un des objets de la présente invention, il est maintenant proposé une composition photoprotectrice comprenant au moins une phase aqueuse, au moins une phase huileuse, au moins un polymère hydrosoluble ou hydrodispersible d'acide acrylamido 2-méthyl propane sulfonique, partiellement ou totalement neutralisé, réticulé ou non-réticulé et au moins un système filtrant les radiations UV, caractérisée par le fait que le système filtrant comprend au moins un filtre UV-A du type 4,4-diarylbutadiène ladite composition se présentant sous forme d'émulsion huile-dans-eau.

D'autres caractéristiques, aspects et avantages de l'invention apparaîtront à la lecture de la description détaillée qui va suivre.

Les polymères utilisés conformément à l'invention sont des homopolymères ou copolymères, réticulés ou non-réticulés comportant au moins le monomère acide acrylamido 2-méthyl propane sufonique (AMPS), sous forme libre ou bien partiellement ou totalement neutralisée.

De façon préférentielle, les polymères d'AMPS conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés » des polymères totalement ou pratiquement totalement neutralisés, c'est-à-dire neutralisés à au moins 90 %.

Ces polymères d'AMPS selon l'invention peuvent être réticulés ou non-réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Les polymères d'AMPS conformes à l'invention peuvent être hydrosolubles ou hydrodispersibles. Ils sont dans ce cas :
- soit des "homopolymères" ne comportant que des monomères AMPS et, s'ils sont réticulés, un ou plusieurs agents de réticulation tels que ceux définis ci-dessus ;
- soit des copolymères obtenus à partir de l'AMPS et d'un ou plusieurs monomères à insaturation éthylénique hydrophiles ou hydrophobes et, s'ils sont réticulés, un ou plusieurs agents de réticulation tels que ceux définis ci-dessus. Lorsque lesdits copolymères comportent des monomères à insaturation éthylénique hydrophobes, ces derniers ne comportent pas de chaîne grasse et sont de préférence présents dans de faibles quantités.

On entend par "chaîne grasse", au sens de la présente invention, toute chaîne hydrocarbonée comportant au moins 7 atomes de carbone.

Par « hydrosoluble ou hydrodispersible », on entend des polymères qui, introduits dans une phase aqueuse à 25°C, à une concentration massique égale à 1%, permettent l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 60%, de préférence d'au moins 70%.

Les polymères d'AMPS conformes à l'invention peuvent être également amphiphiles à savoir qu'ils comportent à la fois une partie hydrophile et une partie hydrophobe comportant au moins une chaîne grasse.

### Polymères hydrosolubles ou hydrodispersibles

Lorsque les polymères conformes à l'invention sont des copolymères hydrosolubles ou hydrodispersibles (non-amphiphiles), ils sont :
- soit des "homopolymères" ne comportant que des monomères AMPS et, s'ils sont réticulés, un ou plusieurs agents de réticulation.
- soit des copolymères obtenus à partir de l'AMPS et d'un ou plusieurs monomères à insaturation éthylénique hydrophiles ou hydrophobes et, s'ils sont réticulés, un ou plusieurs agents de réticulation. Lorsque lesdits copolymères comportent des monomères à insaturation éthylénique hydrophobes ces derniers ne comportent pas de chaîne grasse (nombre d'atomes carbone ne dépassant pas 6)

Les "homopolymères" selon l'invention sont de préférence réticulés et neutralisés, et ils peuvent être obtenus selon le procédé de préparation comprenant les étapes suivantes :
(a) on disperse ou on dissout le monomère tel que l'AMPS sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Comme polymères de ce type, on peut citer notamment l'homopolymère réticulé et neutralisé d'acide 2-acrylamido 2-méthylpropane sulfonique, commercialisé par la société Hoechst sous la dénomination commerciale « Hostacerin AMPS » (nom CTFA: ammonium polyacryldimethyltauramide).

Les copolymères hydrosolubles ou hydrodispersibles d'AMPS selon l'invention contiennent des monomères à insaturation éthylénique hydrosolubles, des monomères hydrophobes ou leurs mélanges.

Les comonomères hydrosolubles peuvent être ioniques ou non-ioniques.

Parmi les comonomères hydrosolubles ioniques, on peut citer par exemple les composés suivants et leurs sels :
- l'acide (méth)acrylique,
- l'acide styrène sulfonique,
- l'acide vinylsulfonique et l'acide (méth)allylsulfonique,
- l'acide vinyl phosphonique,
- l'acide maléique,
- l'acide itaconique,
- l'acide crotonique,
- le chlorure de diméthyldiallyl ammonium;
- le chlorure de méthylvinylimidazolium,
- les carboxybétaïnes ou sulfobétaïnes éthyléniques obtenues par exemple par quaternisation de monomères à insaturation éthyléniques comportant une fonction amine, par des sels de sodium d'acide carboxylique à halogène mobile (ex : chloroacétate) ou par des sulfones cycliques (ex : propane sulfone).
- les monomères vinyliques hydrosolubles de formule (A) suivante : 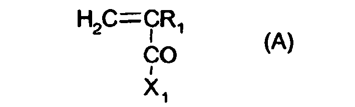 dans laquelle :
   - R₁ est choisi parmi H, -CH₃, -C₂H₅ ou -C₃H₇
   - X₁ est choisi parmi :
      - les oxydes d'alkyle de type -OR₂ où R₂ est un radical hydrocarboné, linéaire ou ramifié, saturé ou insaturé, ayant de 1 à 6 atomes de carbone, substitué par au moins un groupement sulfonique (-SO₃⁻) et/ou sulfate (-SO₄⁻) et/ou phosphate (-PO₄H₂⁻) et/ou un ammonium quaternaire (-N⁺R₃R₄R₅) avec R₃, R₄ et R₅ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₂ + R₃ + R₄ ne dépasse pas 6. Le radical R₁ est éventuellement substitué par un atome d'halogène (iode, brome, chlore, fluor); hydroxy (-OH); éther (-O-), amine primaire (-NH₂); amine secondaire (-NHR₆), tertiaire (-NR₆R₇) avec R₆ et R₇ étant, indépendamment l'un de l'autre, un radical hydrocarboné, linéaire ou ramifié, saturé ou insaturé ayant 1 à 6 atomes de carbone, sous réserve que la somme des atomes de carbone de R₂ + R₆ + R₇ ne dépasse pas 6. Citons le méthacrylate de diméthylaminoéthyle quaternisé (MADAME).
      - les groupements -NH₂, -NHR₈ et -NR₈R₉ dans lesquels R₈ et R₉ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₈+ R₉ ne dépasse pas 6, lesdits R₈ et/ou R₉ étant substitués par au moins un groupement sulfonique (-SO₃⁻) et/ou sulfate (-SO₄⁻) et/ou phosphate (-PO₄H₂⁻) et/ou amine quaternaire (-N⁺R₁₀R₁₁R₁₂) avec R₁₀, R₁₁ et R₁₂ étant, indépendamment l'un de l'autre, un radical hydrocarboné, linéaire ou ramifié, saturé ou insaturé ayant 1 à 6 atomes de carbone, sous réserve que la somme des atomes de carbone de R₈ + R₉ + R₁₀+ R₁₁ + R₁₂ ne dépasse pas 6. Les radicaux R₈ et/ou R₉ sont éventuellement substitués par un atome d'halogène (iode, brome, chlore, fluor); un groupement hydroxy (-OH); éther (-O-), amine primaire (-NH₂); amine secondaire (-NHR₁₃), tertiaire (-NR₁₃R₁₄) avec R₁₃ et R₁₄ étant, indépendamment l'un de l'autre, un radical hydrocarboné, linéaire ou ramifié, saturé ou insaturé ayant 1 à 6 atomes de carbone, sous réserve que la somme des atomes de carbone de R₈ + R₉+ R₁₃ + R₁₄ ne dépasse pas 6 ; Citons le chlorure de (méth)acrylamido propyl triméthyl ammonium (APTAC et MAPTAC).

Parmi, les comonomères hydrosolubles non-ioniques , on peut citer par exemple :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'alcool vinylique de formule CH₂=CHOH,
- les monomères vinyliques hydrosolubles de formule (B) suivante : 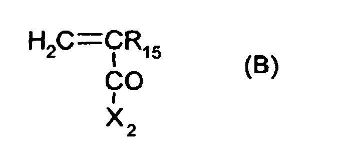 dans laquelle :
   - R₁₅ est choisi parmi H, -CH₃, -C₂H₅ ou -C₃H₇
   - X₂ est choisi parmi :
      - les oxydes d'alkyle de type -OR₁₆ où R₁₆ est un radical hydrocarboné, linéaire ou ramifié, saturé ou insaturé, ayant de 1 à 6 carbones, éventuellement substitué par un atome d'halogène (iode, brome, chlore, fluor); un groupement hydroxy (-OH); éther (-O-), amine primaire (-NH₂); amine secondaire (-NHR₁₇), tertiaire (-NR₁₇R₁₈) avec R₁₇ et R₁₈ étant, indépendamment l'un de l'autre, un radical hydrocarboné, linéaire ou ramifié, saturé ou insaturé ayant 1 à 6 atomes de carbone, sous réserve que la somme des atomes de carbone de R₁₆ + R₁₇ + R₁₈ ne dépasse pas 6 ; Citons par exemple le (méth)acrylate de glycidyle, le méthacrylate d'hydroxyéthyle, et les (méth)acrylates d'éthylène glycol, de diéthylèneglycol ou de polyalkylèneglycol.
      - les groupements -NH₂, -NHR₁₉ et -NR₁₉R₂₀ dans lesquels R₁₉ et R₂₀ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₉ + R₂₀ ne dépasse pas 6, lesdits R₁₉ et R₂₀ étant éventuellement substitués par un atome d'halogène (iode, brome, chlore, fluor) ; un groupement hydroxy (-OH); éther (-O-), amine primaire (-NH₂); amine secondaire (-NHR₂₁), tertiaire (-NR₂₁R₂₂) avec R₂₁ et R₂₂ étant, indépendamment l'un de l'autre, un radical hydrocarboné, linéaire ou ramifié, saturé ou insaturé ayant 1 à 6 atomes de carbone, sous réserve que la somme des atomes de carbone de R₁₉ + R₂₀ + R₂₁ + R₂₂ ne dépasse pas 6. Citons le diméthylaminoéthylméthacrylamide.

Parmi les comonomères hydrophobes sans chaîne grasse, on peut citer par exemple :
- le styrène et ses dérivés tel que le 4-butylstyrène, l'alpha méthylstyrène et le vinyltoluène,
- l'acétate de vinyle de formule CH₂=CH-OCOCH₃ ;
- les vinyléthers de formule CH₂=CHOR dans laquelle R est un radical hydrocarboné, linéaire ou ramifié, saturé ou insaturé, ayant de 1 à 6 carbones;
- l'acrylonitrile,
- la caprolactone,
- le chlorure de vinyle et le chlorure de vinylidène,
- les dérivés siliconés, conduisant après polymérisation à des polymères siliconés tels que le méthacryloxypropyltris(triméthylsiloxy)silane et les méthacrylamides siliconés,
- les monomères vinyliques hydrophobes de formule (C) suivante : 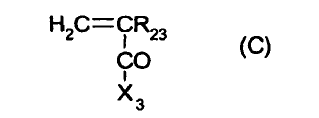 dans laquelle :
   - R₂₃ est choisi parmi H, -CH₃, -C₂H₅ ou -C₃H₇
   - X₃ est choisi parmi :
      - les oxydes d'alkyle de type -OR₂₄ où R₂₄ est un radical hydrocarboné, linéaire ou ramifié, saturé ou insaturé, ayant de 1 à 6 atomes de carbone.
      - les groupements -NH₂, -NHR₂₅ et -NR₂₅R₂₆ dans lesquels R₂₅ et R₂₆ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₂₅+ R₂₆ ne dépasse pas 6. Citons par exemple, le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle.

Les polymères d'AMPS hydrosolubles ou hydrodispersibles de l'invention ont de préférence une masse molaire allant de 50 000 g/mole à 10 000 000 g/mole, de préférence de 80 000 g/mole à 8 000 000 g/mole, et de façon encore plus préférée de 100 000 g/mole à 7 000 000 g/mole.

Comme copolymères hydrosolubles ou hydrodispersibles obtenus à partir d'AMPS et de monomères hydrosolubles à insaturation éthylénique, on peut citer par exemple ceux obtenus à partir d'AMPS et d'acrylamide ou de méthylacrylamide, comme par exemple le copolymère réticulé acrylamide/acrylamido-2-methyl propane sulfonate de sodium en émulsion dans l'isoparaffine en C₁₃-C₁₄ et le laureth-7 (nom CTFA : Polyacrylamide/C₁₃-C₁₄ Isoparaffin/ laureth-7) commercialisé sous la dénomination SEPIGEL 305 ou le copolymère réticulé acrylamide/acrylamido-2-methyl propane sulfonate de sodium en émulsion inverse à 40% dans l'isohexadecane et le polysorbate-80 (nom CTFA : Acrylamide/Sodium Acryloyldimethyltaurate/Isohexadecane/polysorbate-80) commercialisé sous la dénomination SIMULGEL 600 par la société SEPPIC. On peut aussi citer les copolymères d'AMPS et de vinylpyrrolidone ou de vinylformamide, tels que les produits Ammonium Acryloyldimethyltaurate/VP Copolymer (nom INCI) commercialisés sous la dénomination ARISTOFLEX AVC par la société CLARIANT. On peut aussi citer les copolymères d'AMPS et d'acrylate de sodium, comme par exemple le copolymère réticulé AMPS/acrylate de sodium en émulsion inverse dans un mélange eau/isohexadécane/oléate de sorbitane (nom CTFA : Acrylamide/Sodium Acryloyldimethyltaurate/Isohexadecane/polysorbate-80) commercialisé sous la dénomination SIMULGEL EG par la société SEPPIC. On peut citer également les copolymères d'AMPS et d'hydroxyéthyl acrylate, comme par exemple le copolymère réticulé AMPS/hydroxyéthyl acrylate en émulsion inverse dans un mélange polysorbate 60/squalane (nom INCI : Hydroxyethyl acrylate/Sodium Acryloyldimethyltaurate copolymer (and) squalane (and) polysorbate 60) commercialisé sous la dénomination SIMULGEL NS par la société SEPPIC.

### Polymères d'AMPS amphiphiles

Les polymères d'AMPS conformes à l'invention amphiphiles comportent à la fois une partie hydrophile et une partie hydrophobe comportant au moins une chaîne grasse.

La chaîne grasse présente dans les polymères de l'invention comporte de préférence de 7 à 30 atomes de carbone, plus préférentiellement de 7 à 22 atomes de carbone et encore plus préférentiellement de 7 à 18 atomes et plus particulièrement de 12 à 18 atomes de carbones.

Les polymères amphiphiles conformes à l'invention ont en général un poids moléculaire en poids allant de 50 000 à 10 000 000, plus préférentiellement de 100 000 à 8 000 000 et encore plus préférentiellement de 100 000 à 7 000 000.

Les polymères amphiphiles d'AMPS selon l'invention peuvent être réticulés ou non-réticulés. Les agents de réticulation peuvent être choisis parmi ceux cités ci-dessus. On utilisera plus particulièrement le méthylène-bis-acrylamide, le méthacrylate d'allyle ou le triméthylol propane triacrylate (TMPTA). Le taux de réticulation varie de préférence de 0,01 à 10% en moles et plus particulièrement de 0,2 à 2% en moles par rapport au polymère.

Les polymères d'AMPS amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂ tels que ceux décrits dans la demande de brevet WO00/31154. Ces polymères peuvent également contenir d'autres monomères hydrophiles à insaturation éthylénique choisis par exemple parmi l'acide acrylique, l'acide méthacrylique ou leurs dérivés alkyl substitués en β ou leurs esters obtenus avec des mono ou polyalkyleneglycols, l'acrylamide, le méthacrylamide, la vinylpyrrolidone , l'acide itaconique ou l'acide maléique ou leurs mélanges.

Les polymères préférentiels de l'invention sont choisis parmi les polymères amphiphiles d'AMPS et d'au moins un monomère à insaturation éthylénique comportant au moins une partie hydrophobe ayant de 7 à 30 atomes de carbone et plus préférentiellement de 7 à 22 atomes de carbone et encore plus préférentiellement de 7 à 18 atomes de carbone et plus particulièrement de 12 à 18 atomes de carbone. Cette partie hydrophobe peut être un radical alkyle linéaire, saturé ou insaturé (par exemple n-octyle, n-décyle, n-hexadécyle, n-dodécyle, oléyle), ramifié (par exemple isostéarique) ou cyclique (par exemple cyclododécane ou adamantane).

Ces mêmes polymères peuvent contenir en plus un ou plusieurs comonomères hydrophiles à insaturation éthylènique comme l'acide acrylique, l'acide méthacrylique ou leurs dérivés alkylsubstitués en β ou leurs esters obtenus avec des mono ou polyalkylèneglycols, l'acrylamide, le méthacrylamide la vinylpyrrolidone, l'acide itaconique ou l'acide maléique.

Ces mêmes polymères peuvent contenir en plus un ou plusieurs co-monomères hydrophobes à insaturation éthylènique, comprenant par exemple :
- un radical fluoré ou alkylfluoré en C₇-C₁₈ (par exemple le groupement de formule - (CH₂)₂-(CF₂)₉-CF₃)
- un radical cholestéryle ou un radical dérivé de cholestérol (par exemple l'hexanoate de cholestéryle)
- un groupe polycyclique aromatique comme le naphtalène ou le pyrène
- un radical siliconé ou alkylsiliconé ou encore alkylfluorosiliconé.

Ces copolymères sont décrits notamment dans la demande de brevet EP-A-750899, le brevet US-A-5089578 et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Micelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
   « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Ils sont également décrits dans les demandes de brevet (CLARIANT) : EP 1 069 142, WO 02/44224, WO02/44225, WO02/44227, WO02/44229, WO02/44230, WO02/44231, WO02/44267, WO02/44268, WO02/44269, WO02/44270, WO02/44271, WO02/43677, WO02/43686, WO02/43687, WO02/43688, WO02/43689.

Les monomères hydrophobes à insaturation éthylénique de l'invention sont choisis de préférence parmi les acrylates ou les acrylamides de formule (1) suivante : 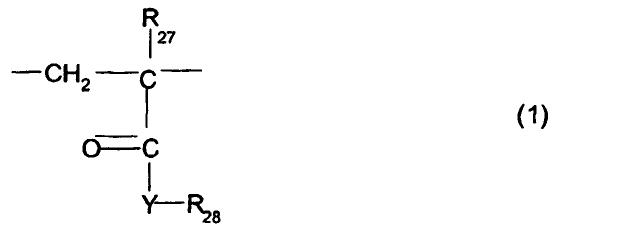 dans laquelle R₂₇ désigne un atome d'hydrogène, un radical alkyle linéaire ou ramifié en C₁-C₆ (de préférence méthyle) ; Y désigne O ou NH ; R₂₈ désigne un radical hydrophobe comportant une chaîne grasse ayant de 7 à 22 atomes de carbone, et de préférence de 7 à 18, et plus particulièrement de 12 à 18 atomes de carbone.

Le radical hydrophobe R₂₈ est choisi de préférence parmi les radicaux alkyles linéaires en C₇-C₁₈, saturés ou insaturés (par exemple n-octyle, n-décyle, n-hexadécyle, n-dodécyle, oléyle), ramifiés (par exemple isostéarique) ou cycliques (par exemple cyclododécane ou adamantane) ; les radicaux alkylperfluorés en C₇-C₁₈ (par exemple le groupement de formule -(CH₂)₂-(CF₂)₉-CF₃); le radical cholestéryle ou un ester de cholestérol comme l'hexanoate de cholestéryle ; les groupes polycycliques aromatiques comme le naphtalène ou le pyrène. Parmi ces radicaux, on préfère plus particulièrement les radicaux alkyles linéaires et ramifiés.

Selon une forme particulièrement préférée de l'invention, le radical hydrophobe R₂₈ comporte en plus au moins un motif oxyde d'alkylène et de préférence une chaîne polyoxyalkylénée. La chaîne polyoxyalkylénée de façon préférentielle est constituée de motifs oxyde d'éthylène et/ou de motifs oxyde de propylène et encore plus particulièrement constituée uniquement de motifs oxyde d'éthylène. Le nombre de moles de motifs oxyalkylénés varie en général de 1 à 30 moles et plus préférentiellement de 1 à 25 moles et encore plus préférentiellement de 3 à 20 moles.

Parmi ces polymères, on peut citer :
- les copolymères réticulés ou non réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans la demande EP-A-750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, par rapport au polymère, tels que ceux décrits dans le brevet US-A-5,089,578.
   Comme polymères amphiphiles, on peut également citer les copolymères d'AMPS totalement neutralisé et de méthacrylate de n-dodécyle, de n-hexadécyle et/ou de n-octadécyle, ainsi que les copolymères d'AMPS et de n-dodécylméthacrylamide, non-réticulés et réticulés.
   On citera plus particulièrement les copolymères amphiphiles réticulés ou non réticulés constitués :
   (a) de motifs acide 2-acrylamido 2-méthylpropane sulfonique (AMPS) de formule (2) suivante : 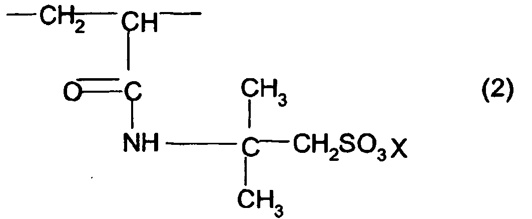 dans laquelle X⁺ est un proton, un cation de métal alcalin, un cation alcalino-terreux ou l'ion ammonium ;
   (b) et de motifs de formule (3) suivante : 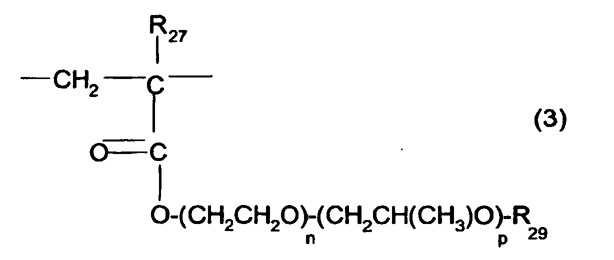
dans laquelle n et p, indépendamment l'un de l'autre désignent un nombre de moles et varie de 0 à 30, de préférence de 1 à 25 et plus préférentiellement de 3 à 20 sous réserve que n + p soit inférieur ou égal à 30, de préférence inférieur à 25 et encore mieux inférieur à 20 ; R₂₇ a la même signification indiquée ci-dessus dans la formule (1) et R₂₉ désigne un alkyle linéaire ou ramifié comportant m atomes de carbone allant de 7 à 22, de préférence de 7 à 18 atomes de carbone et encore mieux de 12 à 18 atomes de carbone.

Dans la formule (2), le cation X⁺ désigne plus particulièrement le sodium ou l'ammonium.

Parmi les monomères de formule (3) on peut citer,
- les esters d'acide (méth)acrylique et d'alcool gras en C₁₀-C₁₈ polyoxethylénés à 8 OE comme le produit GENAPOL C-080 vendu par la Société CLARIANT.
- les esters d'acide (méth)acrylique et d'oxoalcool gras en C₁₁ polyoxyéthyléné à 8 OE comme le produit GENAPOL UD-080 vendu par la Société CLARIANT.
- les esters d'acide (méth)acrylique et d'alcool gras polyoxyéthyléné en C₁₂-C₁₄ à 7 OE comme le produit GENAPOL LA-070 vendu par la Société CLARIANT.
- les esters d'acide (méth)acrylique et d'alcool gras polyoxyéthyléné en C₁₂-C₁₄ à 11 OE comme le produit GENAPOL LA-110 vendu par la Société CLARIANT.
- les esters d'acide (méth)acrylique et d'alcool gras polyoxyéthyléné en C₁₆-C₁₈ à 8 OE comme le produit GENAPOL T-080 vendu par la Société CLARIANT.
- les esters d'acide (méth)acrylique et d'alcool gras polyoxyéthyléné en C₁₆-C₁₈ à 15 OE comme le produit GENAPOL T-150 vendu par la Société CLARIANT.
- les esters d'acide (méth)acrylique et d'alcool gras polyoxyéthyléné en C₁₆-C₁₈ à 11 OE comme le produit GENAPOL T-110 vendu par la Société CLARIANT.
- les esters d'acide (méth)acrylique et d'alcool gras polyoxyéthyléné en C₁₆-C₁₈ à 20 OE comme le produit GENAPOL T-200 vendu par la Société CLARIANT.
- les esters d'acide (méth)acrylique et d'alcool gras polyoxyéthyléné en C₁₆-C₁₈ à 25 OE comme le produit GENAPOL T-250 vendu par la Société CLARIANT.
- les esters d'acide (méth)acrylique et d'alcool gras polyoxyéthyléné en C₁₈-C₂₂ à 25 OE et/ ou d'isoalcool gras polyoxyéthyléné en C₁₆-C₁₈ à 25 OE

On choisira plus particulièrement :
(i) ceux non réticulés pour lesquels p= 0, n = 7 ou 25, R₂₇ désigne méthyle et R₂₉ représente un mélange d'alkyle en C₁₂-C₁₄ ou en C₁₆-C₁₈ ,
(ii) ceux réticulés pour lesquels p = 0, n = 8 ou 25, R₂₇ désigne méthyle et R₂₉ représente un mélange d'alkyle en C₁₆-C₁₈.

Ces polymères sont décrits et synthétisés dans la demande EP1069142. Ces polymères amphiphiles particuliers peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères amphiphiles peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Les polymères conformes à l'invention de façon préférentielle sont neutralisés partiellement ou totalement par une base minérale ou organique telle que celles citées ci-dessus.

La concentration molaire en % des motifs de formule (2) et des motifs de formule (3) dans les polymères amphiphiles selon l'invention varie en fonction de l'application cosmétique souhaitée, de la nature de l'émulsion (huile-dans-eau ou eau-dans-huile) et des propriétés rhéologiques de la formulation recherchées. Elle peut varier entre 0,1 et 99,9 % en moles.

Les polymères d'AMPS amphiphiles selon l'invention peu hydrophobes seront plus appropriées pour l'épaississement et/ou la stabilisation des émulsions huile-dans-eau. La proportion molaire en motifs de formule (3) variera de préférence de 0,1 à 50 %, plus particulièrement de 1 à 25 % et encore plus particulièrement de 3 à 10%.

Les polymères d'AMPS amphiphiles selon l'invention plus hydrophobes seront plus appropriées pour l'épaississement et/ou la stabilisation des émulsions eau-dans-huile. La proportion molaire en motifs de formule (3) variera de préférence de 50,1 à 99,9 %, plus particulièrement de 60 à 95 % et encore plus particulièrement de 65 à 90 %.

La distribution des monomères dans les polymères de l'invention peut être, par exemple, alternée, bloc (y compris multibloc) ou quelconque.

Les polymères d'AMPS conformes à l'invention sont généralement présents dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids de par rapport au poids total de la composition.

Les composés 4,4-diarylbutadiènes conformes à l'invention sont choisis de préférence parmi ceux répondant à la formule (I) suivante : 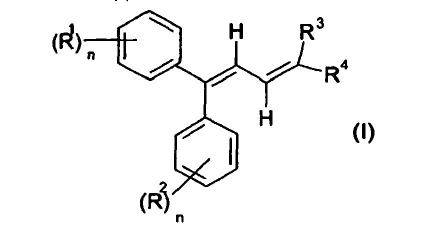 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C_{20,}; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵; COR⁵ ; CONR⁵R⁶ ; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶; CN ; O=S(-R⁶)=O; O=S(-OR⁶)=O ; R⁷O-P-(-OR⁸)=O; un radical alkyle en C₁-C₂₀; un radical alcényle en C₂- C₁₀; un radical cycloalkyle en C₃-C₁₀; un radical bicycloalkyle en C₇-C₁₀; un radical cycloalcényle en C₃-C₁₀; un radical bicycloalcényle en C₇-C₁₀; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀; un radical alcényle en C₂-C₁₀; un radical cycloalkyle en C₃-C₁₀; un radical bicycloalkyle en C₇-C₁₀; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle, 1,1-diméthylbutènyle, 1,2-diméthylbutènyle, 1,3-diméthylbutènyle, 2,2-diméthylbutènyle, 2,3-diméthylbutènyle, 3,3-diméthylbutènyle, 1-éthylbutènyle, 2-éthylbutènyle, 1,1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂, on peut citer : méthoxy, n-propoxy, 1-méthylpropoxy, 1-méthyléthoxy, n-pentoxy, 3-méthylbutoxy, 2,2-diméthylpropoxy, 1-méthyl-1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux alcoxycarbonyle en C₁-C₂₀, on peut citer les esters des alcools en C₁-C₂₀.

Comme radicaux monoalkylamino ou dialkylamino en C₁-C₁₂, on peut citer ceux dont le ou les radicaux alkyle sont choisis parmi méthyle, n-propyle, 2-méthylpropyle, 1,1-diméthyléthyle, hexyle, heptyle, 2-éthylhexyle, isopropyle, 1-méthylpropyle, n-pentyle, 3-méthylbutyle, 2,2-diméthylpropyle, 1-méthyl-1-éthylpropyle, octyle.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) choisis par exemple parmi les halogènes comme chlore, fluor ou brome ; cyano ; nitro ; amino ; C₁-C₄-alkylamino; C₁-C₄ dialkylamino ; Ci-C₄alkyle ; C₁-C₄-alcoxy ; hydroxy ; ils peuvent également comporter de 1 à 3 hétéroatomes comme souffre, oxygène ou azote dont les valences libres peuvent être saturées par un hydrogène ou un radical alkyle en C₁-C₄.

Les groupes bicycloalkyles ou bicycloalcényles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy. On préfère plus particulièrement phényle, méthoxyphényle , naphtyle, thienyle.

Les groupes hétéroaryles comportent en général un ou plusieurs hétéroatomes choisis parmi souffre, oxygène ou azote.

Les groupes hydrosolubilisants sont par exemple des restes carboxy, sulfoxy et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (I) sont connus en eux-mêmes et leurs structures et leurs synthèses sont décrites dans les demandes de brevet DE19755649 , EP916335, EP1133980 et EP1133981.

A titre d'exemple de composé de formule (I), on peut citer les composés suivants :

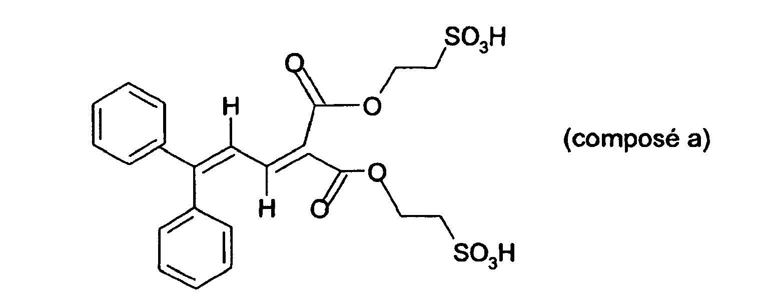

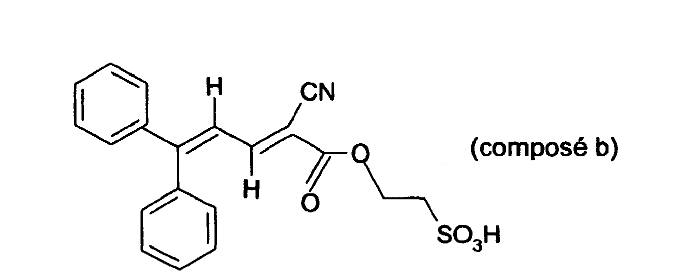

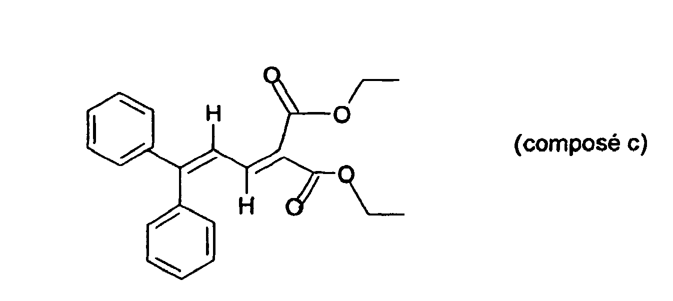

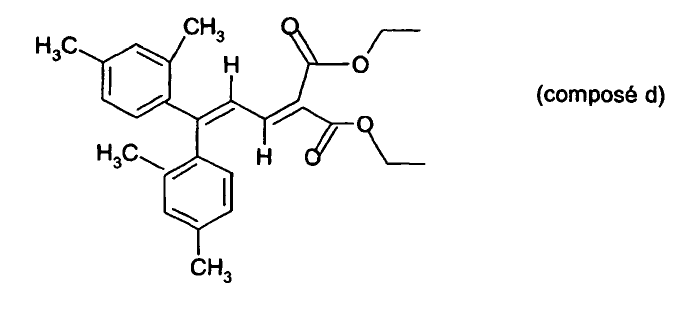

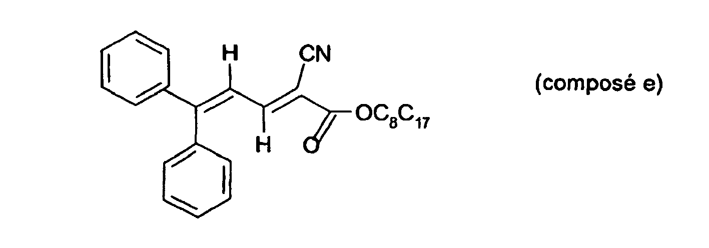

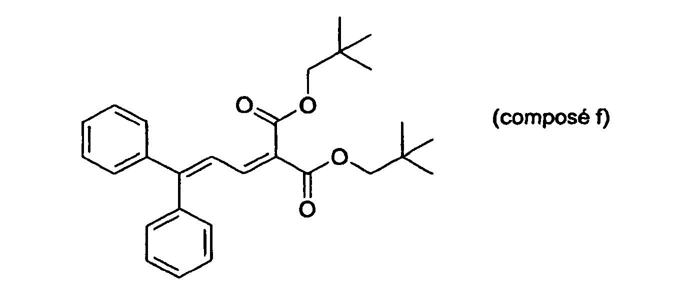

Les composés de formule (I) préférentiels sont ceux pour lesquels
- n = 1 ou 2 ;
- R¹ et R², identiques ou différents, désignent hydrogène, un radical alkyle en C₁-C₂₀,; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵; COR⁵; CONR⁵R⁶ ; un radical alkyle en C₁-C₂₀ ; un radical cycloalkyle en C₃-C₁₀; un radical cycloalcényle en C₃-C₁₀ un radical bicycloalkyle en C₇-C₁₀; phényle, naphtyle ou thienyle éventuellement substitué ;
- R⁴ désigne un groupe COOR⁶; COR⁶ ; CONR⁵R⁶ ; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R⁵ et R⁶, identiques ou différents, désignent hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.
   Parmi ces composés, on préfère plus particulièrement ceux pour lesquels
- R¹ et R², identiques ou différents, désignent hydrogène, un radical alkyle en C₁-C₂₀; un radical alcoxy en C₁-C₂₀; un substituant hydrosolubilisant choisi parmi un groupe carboxylate, un groupe sulfonate ou un reste ammonium ;
- R³ désigne un groupe COOR⁵ ; COR⁵; CONR⁵R⁶;
- R⁴ désigne un groupe COOR⁶ ; COR⁶ ; CONR⁵R⁶ ;
- les radicaux R et R⁶, identiques ou différents, désignent hydrogène ; un radical alkyle en C₁-C₁₂ ; un radical cycloalkyle en C₃-C₆ ; un radical cycloalcényle en C₃-C₁₀; un radical bicycloalkyle en C₇-C₁₀ ; un radical bicycloalcényle en C₃-C₁₀ ; phényle ou naphtyle éventuellement substitué.

Selon un mode particulièrement préféré, les composés de formule (I) sont choisis parmi ceux de formule (I') suivante : 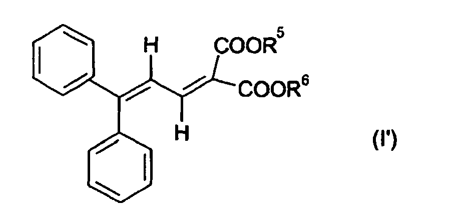 où les radicaux R⁵ et R⁶, identiques ou différents, désignent hydrogène ; un radical alkyle en C₁-C₂₀ ; un radical cycloalkyle en C₃-C₆ ; un radical cycloalcényle en C₃-C₁₀.

Parmi ces composés de formule (I'), on retient plus particulièrement le 1,1-dicarboxy-(2'2'-diméthyl-propyl)-4,4-diphénylbutadiène de structure :

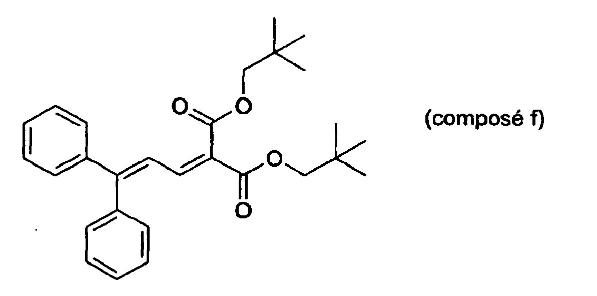

Une autre famille de 4,4-diarylbutadiène pouvant être utilisée dans les émulsions selon l'invention sont ceux répondant à la formule (II) suivante : 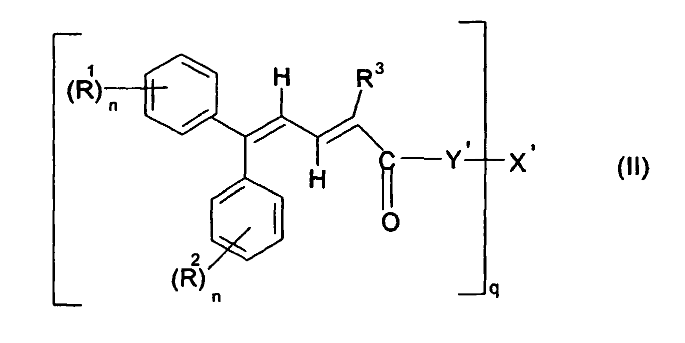 dans laquelle le système diène est de configuration Z,Z ; Z,E ; E,Z ou E,E ou des mélanges desdites configurations et où :
- R¹, R , R³ et n ont les mêmes significations indiquées dans la formule (I) précédente ;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

X' est un reste polyol en C₂-C₂₀ contenant de 2 à 10 groupes hydroxyles et notamment :

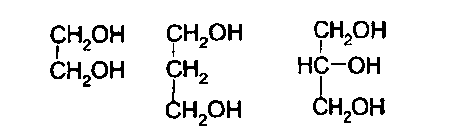

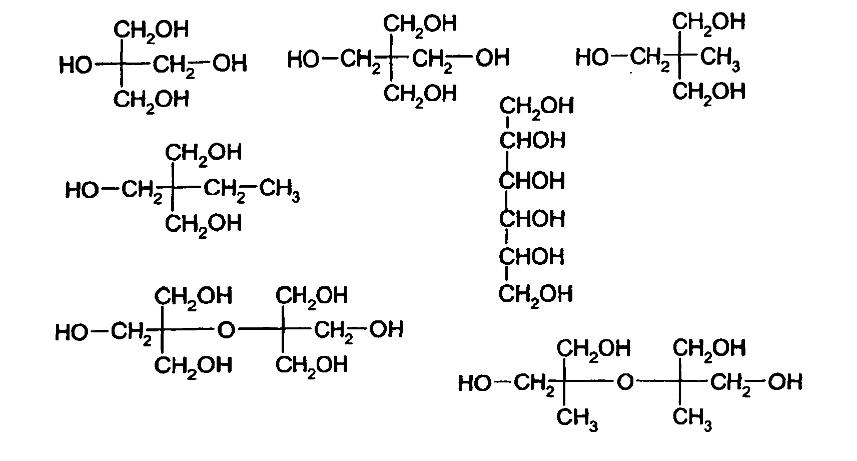

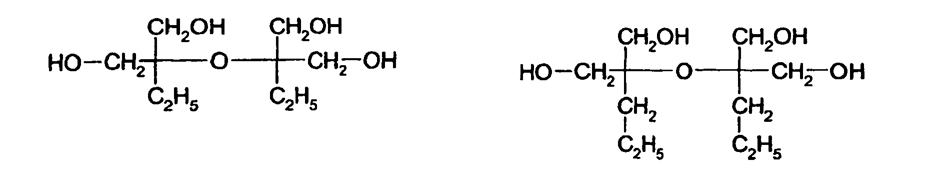

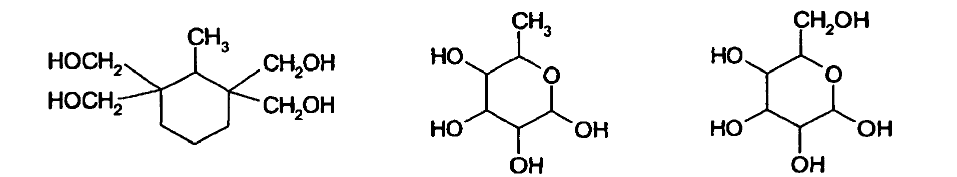

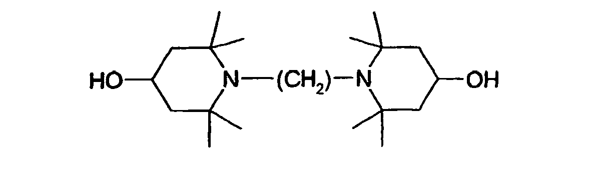

Les composés plus préférentiels de formule (II) sont ceux pour lesquels :
- R¹ et R², identiques ou différents, désignent hydrogène, un radical alkyle en C₁-C₁₂; un radical alcoxy en C₁-C₈ ; un substituant hydrosolubilisant choisi parmi un groupe carboxylate, un groupe sulfonate ou un reste ammonium ;
- R³ désigne un groupe COOR⁵; CONR⁵R⁶ ; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

Les composés encore plus préférentiels de formule (II) sont ceux pour lesquels :
- X' désigne un reste d'éthanol ou de pentaerythrol.

Les composés de formule (II) encore plus particulièrement préférés sont choisis parmi

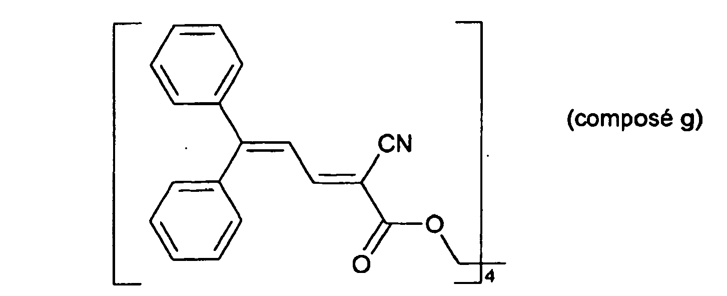

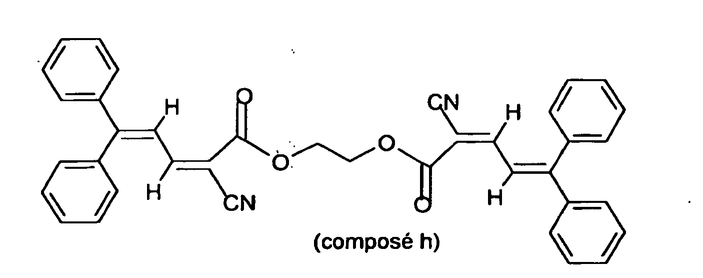

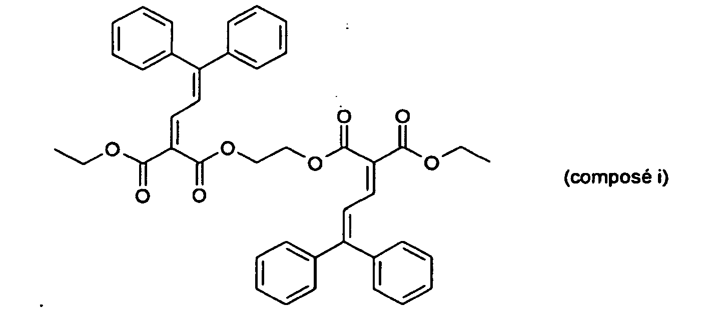

Les composés de formule (II) tels que définis ci-dessus sont connus en eux-mêmes et leurs structures et leurs synthèses sont décrites dans la demande de brevet EP-A-1008586.

Les composés 4,4-diarylbutadiène sont présents de préférence dans la composition dans des proportions allant de 0,1 % à 20% en poids, plus préférentiellement de 1 à 10% en poids par rapport au poids total de la composition.

Les compositions selon l'invention peuvent se présenter sous forme d'émulsions de consistance liquide ou semi-liquide du type lait, obtenues par dispersion d'une phase grasse dans une phase aqueuse (H/E). Ces compositions sont préparées selon les méthodes usuelles.

Selon un mode particulier de réalisation de l'invention, les émulsions huile-dans-eau préparées avec les polymères et/ou copolymères d'AMPS selon l'invention peuvent comporter seulement 1% en poids ou moins, et même être exemptes de tensioactifs émulsionnants, tout en étant stables au stockage.

La nature de la phase grasse rentrant dans la composition des émulsions selon l'invention n'est pas critique et elle peut ainsi être constituée par tous les composés qui sont déja connus de façon générale comme convenant pour la fabrication d'émulsions. En particulier, ces composés peuvent être choisis, seuls ou en mélanges, parmi les différents corps gras, les huiles d'origine végétale, animale ou minérale, les cires naturelles ou synthétiques, et analogues.
Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, les esters comme par exemple le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-a-oléfines.

Comme autres huiles utilisables dans les émulsions selon l'invention, on peut encore citer les benzoates d'alcools gras en C12-C15 (Finsolv TN de FINETEX), les éthers, les dérivés lipophiles d'acide aminé tels que le N-lauroylsarcosinate d'isopropyl (Eldew SL-205 d'Ajinomoto), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés enC10-C18, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, comme par exemple des cires, des gélifiants lipophiles, des tensio-actifs, des particules organiques ou minérales, et notamment ceux qui sont déjà utilisés de manière habituelle dans la fabrication et l'obtention des compositions cosmétiques antisolaires.

De manière classique, la phase aqueuse dispersante peut être constituée par de l'eau, ou un mélange d'eau et d'alcool(s) polyhydrique(s) comme par exemple glycérol, butylène glycol, propylèneglycol et sorbitol, ou bien encore un mélange d'eau et d'alcool(s) inférieur(s) hydrosoluble(s) tels que éthanol, isopropanol ou butanol (solution hydroalcoolique.

Les compositions conformes à l'invention peuvent comporter en plus d'autres filtres UV organiques ou inorganiques complémentaires actifs dans l'UVA et/ou l'UVB, hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; ; les dérivés de l'acide p-aminobenzoïque (PABA) ; les dérivés de benzoxazole tels que décrits dans les demandes de brevet EP0832642, EP1027883, EP1300137 et DE10162844 ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 et leurs mélanges.

Comme exemples de filtres organiques actifs dans l'UV-A et/ou l'UV-B, on peut citer désignés ci-dessous sous leur nom INCI :
Dérivés de l'acide para-aminobenzoique :
   PABA,
   Ethyl PABA,
   Ethyl Dihydroxypropyl PABA,
   Ethylhexyl Diméthyl PABA vendu notamment sous le nom « ESCALOL 507 » par ISP, Glyceryl PABA,
   PEG-25 PABA vendu sous le nom « UVINUL P25 » par BASF,
Dérivés salicyliques :
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LAROCHE,
   Isopropyl Dibenzoylmethane,
Dérivés cinnamiques:
   Ethylhexyl Methoxycinnamate vendu notamment sous le nom commercial « PARSOL MCX » par HOFFMANN LAROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
      - Diisopropyl Methylcinnamate,
   Glyceryl Ethylhexanoate Dimethoxycinnamate
Dérivés de β,β-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF,
   Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF,
   Benzophenone-12,
   le 2-(4-diéthylamino-2-hydroxybenzoyl)-benzoate de n-hexyle
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK ,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
      - Terephthalylidene Dicamphor Sulfonic Acid fabriqué sous le nom « MEXORYL SX » par CHIMEX,
   Polyacrylamidomethyl Benzylidene Camphor fabriqué sous le nom « MESORYL SW » par CHIMEX,
Dérivés de benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par Haarmann et REIMER,
Dérivés de triazine :
   Anisotriazine vendu sous le nom commercial «TINOSORB S » par CIBA SPECIALTY CHEMICALS
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V
   la 2,4,6- tris-(4'amino-benzalmalonate de diisobutyle)-s-triazine.
Dérivés de benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés de benzalmalonate :
   Polyorganosiloxane à fonctions benzalmalonate tel que le polysilicone-15 vendu sous la dénomination commerciale « PARSOL SLX » par HOFFMANN LAROCHE
Dérivés de benzoxazole :
   2,4-bis-[5-1(diméthylpropyl)benzoxazol-2-yl-(4-phenyl)-imino]-6-(2-ethylhexyl)-imino-1,3,5-triazine vendu sous le nom Uvasorb K2A par Sigma 3V ;
   et leurs mélanges.

Les filtres organiques plus particulièrement préférés sont choisis parmi les composés suivants :
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone 15
2,4-bis-[5-1(diméthylpropyl)benzoxazol-2-yl-(4-phenyl)-imino]-6-(2-ethylhexyl)-imino-1,3,5-triazine et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres complémentaires selon l'invention sont généralement présents dans les compositions selon l'invention à une teneur allant de 0,1 % à 30 % en poids et de préférence de 0,5 à 15 % , en poids, par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les actifs, les pigments, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Parmi les solvants organiques, on peut citer les alcools et polyols inférieurs.

Parmi les épaississants on peut citer les polymères acryliques réticulés comme les Carbomers fournis par Novéon, les polymères réticulés acrylates/C10-30 alkylacrylates du type Pemulen fournis par Novéon ou le polyacrylate-3 vendu sous le nom Viscophobe DB 1000 par Amerchol) ; les polymères dérivés de l'acide acrylamido 2-méthylpropane sulfonique (Hostacerin AMPS fourni par Clariant, Sépigel 305 fourni par Seppic), les polymères neutres synthétiques tels que la poly N-vinylpyrrolidone, les polysaccharides comme les gommes de guar, de xanthane et les dérivés cellulosiques modifiés ou non comme la gomme de guar hydroxypropylée, la méthylhydroxyéthylcellulose et l'hydroxypropylméthylcellulose.

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Les compositions cosmétiques selon l'invention peuvent par exemple être utilisées comme produit de soin et/ou de protection solaire pour le visage et/ou le corps , de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

### Exemples

### Exemple A: Crèmes hydratantes et photoprotectrices (Emulsions H/E stabilisées par des tensio-actifs et comprenant un copolymère gélifiant réticulé dérivé d'AMPS)

| **Ingrédients** | **Emulsion 1 (hors invention)** | **Emulsion 2 (invention)** |
|---|---|---|
| Mélange de monostéarate de glycéryl et de stéarate de polyéthylène glycol (100 OE) 50/50 | 2,5 | 2,5 |
| Monostéarate de polyéthylène glycol (50 OE) | 2,5 | 2,5 |
| Alcool stéarylique | 0,5 | 0,5 |
| Octyl-méthoxycinnamate | 7 | 7 |
| 1,1-dicarboxy-(2'2'-diméthyl-propyl)-4,4-diphénylbutadiène (composé f) | 0 | 5 |
| Alkyl C₁₂-₁₅ benzoate | 10 | 10 |
| Glycérine | 3 | 3 |
| Acide téréphtalylidène 3,3'-dicamphosulfonique-10,10' | 5 | 0 |
| Ammonium polyacryldimethyltauramide (HOSTACERIN AMPS) | 1 | 1 |
| Triéthanolamine | 3,55 | 0,01 |
| Conservateur | 0,3 | 0,3 |
| Eau | Qsp 100 | Qsp 100 |
| **Viscosité (Pa.s) sous 200 s⁻¹ (Rhéomat 180)** | 0,16 | 4,7 |

### Mode de préparation:

Les tensio-actifs et éventuellement le dérivé de diarylbutadiène sont solubilisés à 75°C dans la phase huileuse sous agitation; la solution obtenue est macroscopiquement homogène. Chaque émulsion est préparée par introduction lente de la phase huileuse dans la phase aqueuse sous agitation à l'aide d'un homogénéisateur de type Moritz à 70°C sous une vitesse d'agitation de 2000 RPM pendant 15 minutes. Chaque émulsion est ensuite refroidie à la température ambiante sous agitation lente.

Après 24 heures à température ambiante, on observe que l'émulsion 1 devient liquide alors que l'émulsion 2 selon l'invention se présente sous la forme d'une crème brillante, stable et agréable à appliquer sur la peau.

### Exemple B : Laits photoprotecteurs (Emulsions H/E sans tensio-actif stabilisées par un copolymère amphiphile non réticulé dérivé d'AMPS)

| **Ingrédients** | **Emulsion 3 (hors invention)** | **Emulsion 4 (invention)** |
|---|---|---|
| Octyl-méthoxycinnamate | 7 | 7 |
| 1,1-dicarboxy-(2'2'-diméthyl-propyl)-4,4-diphénytbutadiène (composé f) | 0 | 5 |
| Alkyl C₁₂₋₁₅ benzoate | 10 | 10 |
| Glycérine | 3 | 3 |
| Acide téréphtalylidène 3,3'-dicamphosulfonique-10,10' | 5 | 0 |
| Copolymère non réticulé d'AMPS et d'ester d'acide (méth)acrylique et d'alcool gras en C₁₆-C₁₈ polyoxyéthyléné à 8 OE (GENAPOL T-080) tel que celui décrit dans l'exemple 7 de la demande EP1059142 | 0,5 | 0,5 |
| Triéthanolamine | 3,55 | 0,01 |
| Conservateur | 0,3 | 0,3 |
| Eau | qsp 100 | qsp 100 |
| **Viscosité (Pa.s) sous 200 s⁻¹ (Rhéomat 180)** | **0,04** | **0,15** |

### Mode de préparation:

Le copolymère amphiphile d'AMPS est solubilisé pendant 2 heures sous agitation dans la phase aqueuse à 25°C ; la solution obtenue est macroscopiquement homogène. L'émulsion est préparée par introduction lente de la phase huileuse dans la phase aqueuse sous agitation à l'aide d'un homogénéisateur de type Moritz sous une vitesse d'agitation de 2000 RPM pendant 15 minutes.

L'émulsion 3 se fluidifie et perd sa viscosité . L'émulsion 4 selon l'invention reste stable et présente une belle texture de type lait.

### Exemple C : Crèmes photoprotectrices (Emulsions H/E sans tensio-actif stabilisées par un copolymère amphiphile dérivé d'AMPS réticulé)

| **Ingrédients** | **Emulsion 5 (hors invention)** | **Emulsion 6 (invention)** |
|---|---|---|
| Octyl-méthoxycinnamate | 7 | 7 |
| 1,1-dicarboxy-(2'2'-diméthyl-propyl)-4,4-diphénylbutadiène (composé f) | 0 | 5 |
| Alkyl C₁₂₋₁₅ benzoate | 10 | 10 |
| Glycérine | 3 | 3 |
| Acide terephtalylidène 3,3'-dicamphosulfonique-10,10' | 5 | 0 |
| Copolymère réticulé d'AMPS et d'ester d'acide (méth)acrylique et d'alcool gras en C₁₆-C₁₈ polyoxyéthyléné à 25 OE (GENAPOL T-250) tel que celui décrit dans l'exemple 3 de la demande EP1059142 | 0,5 | 0,5 |
| triéthanolamine | 3,55 | 0,01 |
| Conservateur | 0,3 | 0,3 |
| Eau | qsp 100 | qsp 100 |
| **Viscosité (Pa.s) sous 200 s⁻¹ (Rhéomat 180)** | 0,05 | 0,70 |

### Mode de préparation:

Le copolymère amphiphile d'AMPS est solubilisé pendant 2 heures sous agitation dans la phase aqueuse à 25°C ; la solution obtenue est macroscopiquement homogène. Chaque émulsion est préparée par introduction lente de la phase huileuse dans la phase aqueuse sous agitation à l'aide d'un homogénéisateur de type Moritz sous une vitesse d'agitation de 2000 RPM pendant 15 minutes.

L'émulsion 5 se déstabilise macroscopiquement après 24 heures à la température ambiante, avec l'apparition d'un phénomène de crémage. L'émulsion 6 reste stable et présente une belle texture de type lait.

## Revendications

1. Composition photoprotectrice comprenant au moins une phase aqueuse, au moins une phase huileuse, au moins un polymère hydrosoluble ou hydrodispersible d'acide acrylamido 2-méthyl propane sulfonique (AMPS), partiellement ou totalement neutralisé, réticulé ou non-réticulé et au moins un système filtrant les radiations UV, **caractérisée par le fait que** le système filtrant comprend au moins un filtre UV-A du type 4,4-diarylbutadiène ; ladite composition se présentant sous forme d'émulsion huile-dans-eau.

2. Composition selon la revendication 1, **caractérisée en ce que** le polymère d'AMPS est neutralisé partiellement ou totalement par une base minérale ou organique.

3. Composition selon la revendication 2, **caractérisée en ce que** la base minérale est choisie parmi la soude, potasse ou ammoniaque.

4. Composition selon la revendication 2, **caractérisée en ce que** la base organique est choisie parmi la mono-, di- ou tri-éthanolamine, l'aminométhylpropanediol, la N-méthyl-glucamine, les acides aminés basiques et leurs mélanges.

5. Composition selon l'une quelconque des revendications 1 à 4, où le polymère d'AMPS est neutralisé à au moins 90 %.

6. Composition selon l'une quelconque des revendications 1 à 5, où le polymère d'AMPS est réticulé et où l'agent de réticulation est choisi parmi les composés à polyinsaturation oléfinique couramment utilisés pour la réticulation des polymères obtenus par polymérisation radicalaire.

7. Composition selon la revendication 6, où l'agent de réticulation est choisi parmi le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou leurs mélanges.

8. Composition selon la revendication 6, où l'agent de réticulation est choisi parmi le méthylène-bis-acrylamide, le méthacrylate d'allyle ou le triméthylol propane triacrylate (TMPTA).

9. Composition selon l'une quelconque des revendications 1 à 8, où le taux de réticulation va en général de 0,01 à 10 % en mole et plus particulièrement de 0,2 à 2 % en mole par rapport au polymère.

10. Composition selon l'une quelconque des revendications 1 à 9, **caractérisée par le fait que** le polymère d'AMPS est hydrosoluble ou hydrodispersible et choisi dans le groupe constitué par :
(i) les homopolymères d' AMPS réticulés ou non réticulés ;
(ii) les copolymères réticulés ou non réticulés, obtenus à partir de l'AMPS et d'un ou plusieurs monomères à insaturation éthylénique hydrophiles ou monomères à insaturation éthylénique hydrophobes ne comportant pas de chaîne grasse.

11. Composition selon la revendication 10, où les polymères d'AMPS hydrosolubles ou hydrodispersibles ont une masse molaire allant de 50 000 g/mole à 10 000 000 g/mole, de préférence de 80 000 g/mole à 8 000 000 g/mole, et de façon encore plus préférée de 100 000 g/mole à 7 000 000 g/mole.

12. Composition selon la revendication 10 ou 11, où l'homopolymère d' AMPS est l' Ammonium Polyacryloyldimethyltauramide.

13. Composition selon la revendication 10 ou 11, où les copolymères réticulés ou non réticulés, d'AMPS et d'un ou plusieurs monomères à insaturation éthylénique hydrophiles ou hydrophobes sont choisis parmi
(a) les copolymères d'AMPS et d'acrylamide ou de méthylacrylamide ;
(b) les copolymères d'AMPS et de vinylpyrrolidone ou de vinylformamide.

14. Composition selon la revendication 13, où le copolymère hydrosoluble ou hydrodispersible d'AMPS est choisi parmi :
- Polyacrylamide/C₁₃-C₁₄ Isoparaffin/ laureth-7
- Acrylamide/Sodium Acryloyldimethyltaurate/Isohexadecane/polysorbate-80 ;
- Ammonium Acryloyldimethyltaurate/VP Copolymer.

15. Composition selon l'une quelconque des revendications 1 à 9, **caractérisée par le fait que** le polymère d'AMPS est amphiphile.

16. Composition selon la revendication 15, où le polymère d'AMPS amphiphile comporte au moins une chaîne grasse comportant de 7 à 30 atomes de carbone, plus préférentiellement de 7 à 22 atomes de carbone et encore plus préférentiellement de 7 à 18 atomes de carbone et plus particulièrement de 12 à 18 atomes de carbone.

17. Composition selon la revendication 15 ou 16, où le polymère d'AMPS amphiphile a un poids moléculaire en poids allant de 50 000 à 10 000 000, plus préférentiellement de 100 000 à 8 000 000 et encore plus préférentiellement de 100 000 à 7 000 000.

18. Composition selon l'une quelconque des revendications 15 à 17, où les polymères d'AMPS amphiphiles sont choisis parmi les polymères amphiphiles statistiques d'AMPS modifiés par réaction avec une n-monoalkylamine ou une di-n-alkylamine en C₆-C₂₂ et pouvant contenir un ou plusieurs monomères hydrophiles à insaturation éthylénique.

19. Composition selon l'une quelconque des revendications 15 à 17, où les polymères d'AMPS amphiphiles sont choisis parmi les polymères d'AMPS et d'au moins un monomère à insaturation éthylénique comportant au moins une partie hydrophobe ayant de 7 à 30 atomes de carbone et plus préférentiellement de 7 à 22 atomes de carbone et encore plus préférentiellement de 7 à 18 atomes de carbone et plus particulièrement de 12 à 18 atomes de carbone et éventuellement un ou plusieurs co-monomères hydrophiles à insaturation éthylènique.

20. Composition selon la revendication 19, où les monomères à insaturation éthylénique comportant au moins une partie hydrophobe ayant de 7 à 30 atomes de carbone sont choisis parmi les acrylates ou les acrylamides de formule (1) suivante : 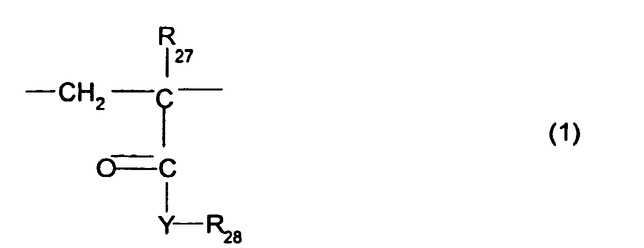 dans laquelle R₂₇ désigne un atome d'hydrogène, un radical alkyle linéaire ou ramifié en C₁-C₆ (de préférence méthyle) ; Y désigne O ou NH ; R₂₈ désigne un radical hydrophobe comportant une chaîne grasse ayant de 7 à 22 atomes de carbone, et de préférence de 7 à 18, et plus particulièrement de 12 à 18 atomes de carbone.

21. Composition selon la revendication 20, où le radical hydrophobe R₂₈ est choisi parmi les radicaux alkyles en C₇-C₁₈ linéaires ou ramifiés, saturés ou insaturés ; les radicaux alkylperfluorés en C₇-C₁₈; le radical cholestéryle ou un ester de cholestérol; les groupes polycycliques aromatiques.

22. Composition selon la revendication 20 ou 21, où le radical hydrophobe R₂₈ comporte en plus au moins un motif oxyde d'alkylène et de préférence une chaîne polyoxyalkylénée.

23. Composition selon la revendication 22, où le nombre de moles de motifs oxyalkylénés varie de 1 à 30 moles et plus préférentiellement de 1 à 25 moles et encore plus préférentiellement de 3 à 20 moles.

24. Composition selon l'une quelconque des revendications 20 à 23, où les polymères d'AMPS amphiphiles sont des copolymères amphiphiles constitués :
(a) de motifs acide 2-acrylamido 2-méthylpropane sulfonique (AMPS) de formule (2) suivante : 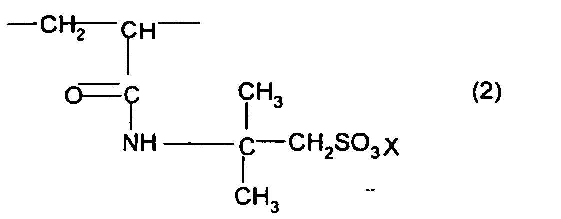 dans laquelle X⁺ est un proton, un cation de métal alcalin, un cation alcalino-terreux ou l'ion ammonium ;
(b) et de motifs de formule (3) suivante : 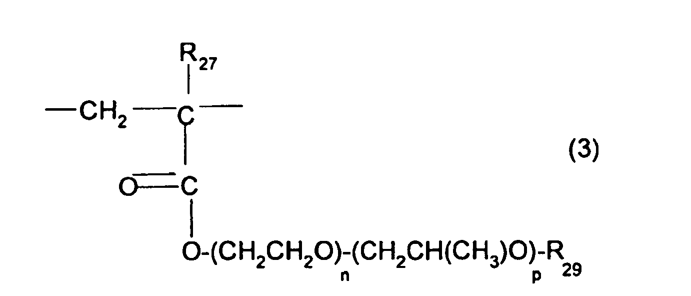
dans laquelle n et p, indépendamment l'un de l'autre désignent un nombre de moles et varie de 0 à 30, de préférence de 1 à 25 et plus préférentiellement de 3 à 20 sous réserve que n + p soit inférieur ou égal à 30, de préférence inférieur à 25 et encore mieux inférieur à 20 ; R₂₇ a la même signification indiquée ci-dessus dans la formule (1) et R₂₉ désigne un alkyle linéaire ou ramifié comportant m atomes de carbone allant de 7 à 22, de préférence de 7 à 18 atomes de carbone et encore mieux de 12 à 18 atomes de carbone.

25. Composition selon la revendication 24, où X⁺ désigne le sodium ou l'ammonium.

26. Composition selon la revendication 24 ou 25, où les polymères d'AMPS amphiphiles sont choisis parmi :
(i) ceux non réticulés pour lesquels p= 0 , n = 7 ou 25, R₂₇ désigne méthyle et R₂₉ représente un mélange d'alkyle en C₁₂-C₁₄ ou en C₁₆-C₁₈ ,
(ii) ceux réticulés pour lesquels p = 0, n = 8 ou 25, R₂₇ désigne méthyle et R₂₉ représente un mélange d'alkyle en C₁₆-C₁₈.

27. Composition selon l'une quelconque des revendications 24 à 26, où la proportion molaire en motifs de formule (3) varie de 0,1 à 50 %, plus particulièrement de 1 à 25 % et encore plus particulièrement de 3 à 10%.

28. Composition selon l'une quelconque des revendications 24 à 26, où la proportion molaire en motifs de formule (3) varie de 50,1 à 99,9 % et plus particulièrement de 60 à 95 % et encore plus particulièrement de 65 à 90 %.

29. Composition selon l'une quelconque des revendications 1 à 28, où les polymères d'AMPS sont présents dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids par rapport au poids total de la composition.

30. Composition selon l'une quelconque des revendications 1 à 29, où le filtre UV-A du type 4,4-diarylbutadiène répond à la formule (I) suivante : 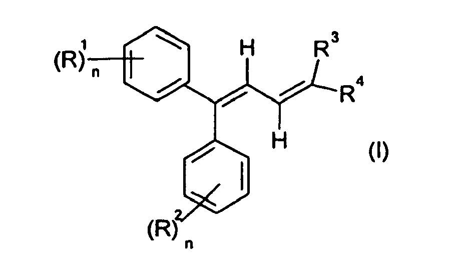 dans laquelle le système diène est de configuration Z,Z ; Z,E ; E,Z ou E,E ou des mélanges desdites configurations et où :
- R¹ et R², identiques ou différents, désignent l'hydrogène, un radical alkyle en C1-C20,; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵; COR⁵ ; CONR⁵R⁶; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶ ; CN ; O=S(-R⁶)=O; O=S(-OR⁶)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀; un radical bicycloalcényle en C₇-C₁₀; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀ ; un radical cycloalkyle en C₃-C₁₀ ; un radical bicycloalkyle en C₇-C₁₀ ; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

31. Composition selon la revendication 30, où le composé de formule (I) est choisi parmi ceux pour lesquels
- n = 1 ou 2 ;
- R¹ et R², identiques ou différents, désignent l'hydrogène, un radical alkyle en C₁-C₂₀ ; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵; COR⁵; CONR⁵R⁶; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶; COR⁶; CONR⁵R⁶ ; un radical alkyle en C₁-C₂₀ ; un radical cycloalkyle en C₃-C₆ ; un radical cycloalcényle en C₃-C₁₀; un radical bicycloalkyle en C₇-C₁₀ ; phényle, naphtyle ou thienyle éventuellement substitué ;
- les radicaux R et R⁶, identiques ou différents, désignent l'hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

32. Composition selon la revendication 31, où le composé de formule (I) est choisi parmi ceux pour lesquels
- R¹ et R², identiques ou différents, désignent l'hydrogène, un radical alkyle en C₁-C₂₀ ; un radical alcoxy en C₁-C₂₀ ; un substituant hydrosolubilisant choisi parmi un groupe carboxylate, un groupe sulfonate ou un reste ammonium ;
- R³ désigne un groupe COOR⁵; COR⁵; CONR⁵R⁶;
- R⁴ désigne un groupe COOR⁶ ; COR⁶; CONR⁵R⁶;
- les radicaux R et R⁶, identiques ou différents, désignent l'hydrogène ; un radical alkyle en C₁-C₁₂; un radical cycloalkyle en C₃-C₆; un radical cycloalcényle en C₃-C₁₀; un radical bicycloalkyle en C₇-C₁₀; un radical bicycloalcényle en C₃-C₁₀ ; phényle ou naphtyle éventuellement substitué.

33. Composition selon la revendication 32, où le composé de formule (I) est choisi parmi ceux de formule (I') suivante : 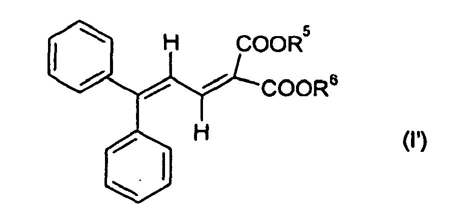 où les radicaux R⁵ et R⁶, identiques ou différents, désignent l'hydrogène; un radical alkyle en C₁-C₂₀ ; un radical cycloalkyle en C₃-C₆ ; un radical cycloalcényle en C₃-C₁₀.

34. Composition selon la revendication 33, où le composé de formule (I') est le 1,1-dicarboxy(2'2'-dimethyl-propyl)-4,4-diphenylbutadiene de structure :

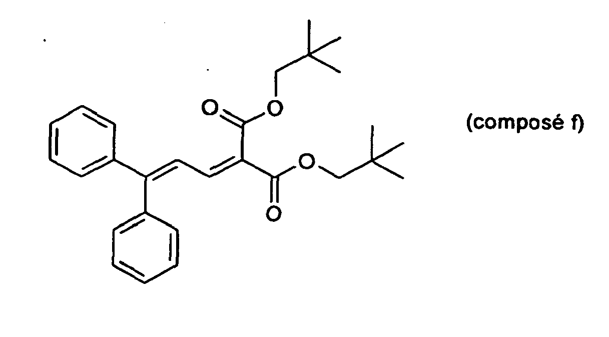

35. Composition selon l'une quelconque des revendications 1 à 29, où le filtre UV-A du type 4,4-diarylbutadiène répond à la formule (II) suivante : 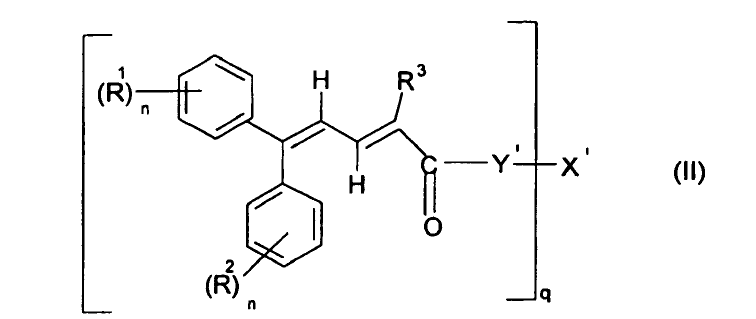 dans laquelle le système diène est de configuration Z,Z ; Z,E ; E,Z ou E,E ou des mélanges desdites configurations et où :
- R¹, R², R³ et n ont les mêmes significations indiquées dans la formule (I) telle que définie dans la revendication 30;
- Y' désigne un groupe -O- ou -NR⁹-
- R⁹ désigne l'hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

36. Composition selon la revendication 35, où le composé de formule (II) est choisi parmi ceux pour lesquels :
- R¹ et R², identiques ou différents, désignent l'hydrogène, un radical alkyle en C₁-C₁₂; un radical alcoxy en C₁-C₈; un substituant hydrosolubilisant choisi parmi un groupe carboxylate, un groupe sulfonate ou un reste ammonium ;
- R³ désigne un groupe COOR⁵; CONR⁵R⁶ ; CN ; un radical cycloalkyle en C₃-C₁₀; un radical bicycloalkyle en C₇-C₁₀;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

37. Composition selon la revendication 36, où le composé de formule (II) est choisi parmi ceux pour lesquels X' désigne un reste d'éthanol ou de pentaerythritol.

38. Composition selon la revendication 37, où le composé de formule (II) est choisi parmi les composés suivants :

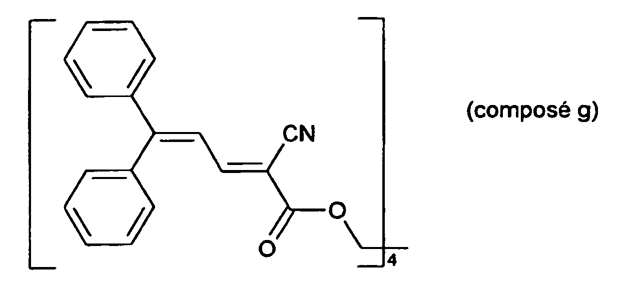

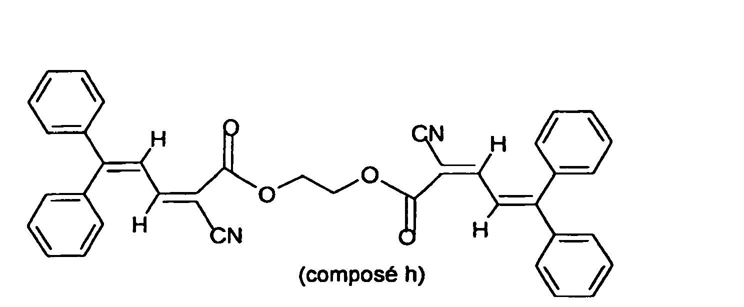

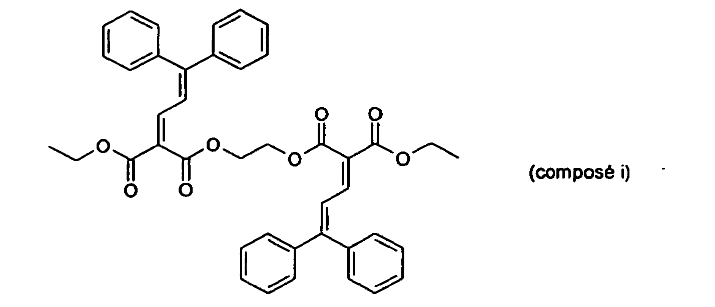

39. Composition selon l'une quelconque des revendications 1 à 38, **caractérisée en ce que** le ou les composés 4,4-diarylbutadiène sont présents dans des proportions allant de 0,1 % à 20% en poids, plus préférentiellement de 1 à 10% en poids par rapport au poids total de l'émulsion.

40. Composition selon l'une quelconque des revendications 1 à 39, **caractérisée en ce qu'**elle contient en outre au moins un filtre solaire organique ou inorganique complémentaire actif dans l'UV-A et/ou l'UV-B, hydrosolubles, liposolubles ou bien insolubles dans les solvants cosmétiques couramment utilisés.

41. Composition selon la revendication 40, ou les filtres organiques complémentaires sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrylate; les

42. Composition selon la revendication 41, où les filtres organiques complémentaires sont choisis parmi
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
2,4-bis-[5-1(diméthylpropyl)benzoxazol-2-yl-(4-phenyl)-imino]-6-(2-ethylhexyl)-imino-1,3,5-triazine et leurs mélanges.

43. Composition selon la revendication 40, où les filtres complémentaires inorganiques sont choisis parmi des pigments ou des nanopigments d'oxydes métalliques, enrobés ou non.

44. Composition selon la revendication 43, où les filtres complémentaires inorganiques sont des nanopigments d'oxyde de titane, amorphe ou cristallisé, sous forme rutile et/ou anatase, de fer, de zinc, de zirconium ou de cérium

45. Composition selon l'une quelconque des revendications 1 à 44, **caractérisée en ce qu'**elle contient en outre au moins un agent de bronzage et/ou de brunissage artificiel de la peau.

46. Composition selon l'une quelconque des revendications 1 à 45, **caractérisée en ce qu'**elle contient en outre au moins un adjuvant cosmétique choisi parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les actifs, les pigments, les polymères, les propulseurs, les agents alcalinisants ou acidifiants ou tout autre ingrédient habituellement utilisé dans le domaine cosmétique et/ou dermatologique.

47. Composition selon l'une quelconque des revendications 1 à 46, **caractérisée en ce qu'**elle se présente sous forme d'émulsion huile-dans-eau comportant au plus 1% en poids par rapport au poids total de la composition en tensioactif émulsionnant.

48. Utilisation d'une composition telle que définie selon l'une quelconque des revendications 1 à 47 48 pour la fabrication de produits pour le traitement cosmétique de la peau, des lèvres et des cheveux, y compris le cuir chevelu, en particulier pour la protection et/ou le soin de la peau, des lèvres et/ou des cheveux et/ou pour le maquillage de la peau et/ou des lèvres.

## Claims

1. Photoprotective composition comprising at least one aqueous phase, at least one oily phase, at least one partially or completely neutralized, crosslinked or non-crosslinked water-soluble or water-dispersible acrylamido-2-methylpropanesulphonic acid (AMPS) polymer and at least one UV radiation-screening system, **characterized in that** the screening system comprises at least one UV-A-screening agent of the 4,4-diarylbutadiene type; the said composition being provided in the form of an oil-in-water emulsion.

2. Composition according to Claim 1, **characterized in that** the AMPS polymer is partially or completely neutralized with an inorganic or organic base.

3. Composition according to Claim 2, **characterized in that** the inorganic base is chosen from sodium hydroxide, potassium hydroxide or aqueous ammonia.

4. Composition according to Claim 2, **characterized in that** the organic base is chosen from mono-, di- or triethanolamine, aminomethylpropanediol, N-methylglucamine, basic amino acids and mixtures thereof.

5. Composition according to any one of Claims 1 to 4, where the AMPS polymer is at least 90% neutralized.

6. Composition according to any one of Claims 1 to 5, where the AMPS polymer is crosslinked and where the crosslinking agent is chosen from the olefinically polyunsaturated compounds commonly used for the crosslinking of polymers obtained by free-radical polymerization.

7. Composition according to Claim 6, where the crosslinking agent is chosen from divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allyl ethers of alcohols of the sugar series, or other allyl or vinyl ethers of polyfunctional alcohols, and the allyl esters of phosphoric and/or vinylphosphonic acid derivatives, or mixtures thereof.

8. Composition according to Claim 6, where the crosslinking agent is chosen from methylenebisacrylamide, allyl methacrylate or trimethylolpropane triacrylate (TMPTA).

9. Composition according to any one of Claims 1 to 8, where the degree of crosslinking ranges in general from 0.01 to 10 mol% and more particularly from 0.2 to 2 mol% relative to the polymer.

10. Composition according to any one of Claims 1 to 9, **characterized in that** the AMPS polymer is water-soluble or water-dispersible and is chosen from the group consisting of:
(i) crosslinked or non-crosslinked AMPS homopolymers;
(ii) crosslinked or non-crosslinked copolymers obtained from AMPS and from one or more hydrophilic ethylenically unsaturated monomers or hydrophobic ethylenically unsaturated monomers containing no fatty chain.

11. Composition according to Claim 10, where the water-soluble or water-dispersible AMPS polymers have a molar mass ranging from 50 000 g/mol to 10 000 000 g/mol, preferably from 80 000 g/mol to 8 000 000 g/mol, and more preferably still from 100 000 g/mol to 7 000 000 g/mol.

12. Composition according to Claim 10 or 11, where the AMPS homopolymer is ammonium polyacryloyldimethyltauramide.

13. Composition according to Claim 10 or 11, where the crosslinked or non-crosslinked copolymers of AMPS and of one or more hydrophilic or hydrophobic ethylenically unsaturated monomers are chosen from:
(a) copolymers of AMPS and acrylamide or methylacrylamide;
(b) copolymers of AMPS and vinylpyrrolidone or vinylformamide.

14. Composition according to Claim 13, where the water-soluble or water-dispersible copolymer of AMPS is chosen from:
- polyacrylamide/C₁₃-C₁₄ isoparaffin/laureth-7
- acrylamide/sodium acryloyldimethyltaurate/isohexadecane/polysorbate-80;
- ammonium acryloyldimethyltaurate/VP copolymer.

15. Composition according to any one of Claims 1 to 9, **characterized in that** the AMPS polymer is amphiphilic.

16. Composition according to Claim 15, where the amphiphilic AMPS polymer contains at least one fatty chain containing from 7 to 30 carbon atoms, more preferably from 7 to 22 carbon atoms and more preferably still from 7 to 18 carbon atoms and more particularly from 12 to 18 carbon atoms.

17. Composition according to Claim 15 or 16, where the amphiphilic AMPS polymer has a weight-average molecular weight ranging from 50 000 to 10 000 000, more preferably from 100 000 to 8 000 000 and more preferably still from 100 000 to 7 000 000.

18. Composition according to any one of Claims 15 to 17, where the amphiphilic AMPS polymers are chosen from random amphiphilic AMPS polymers modified by reaction with a C₆-C₂₂ n-monoalkylamine or di-n-alkylamine and which may contain one or more ethylenically unsaturated hydrophilic monomers.

19. Composition according to any one of Claims 15 to 17, where the amphiphilic AMPS polymers are chosen from AMPS polymers and at least one ethylenically unsaturated monomer containing at least one hydrophobic part having from 7 to 30 carbon atoms and more preferably from 7 to 22 carbon atoms and more preferably still from 7 to 18 carbon atoms and more particularly from 12 to 18 carbon atoms and optionally one or more ethylenically unsaturated hydrophilic comonomers.

20. Composition according to Claim 19, where the ethylenically unsaturated monomers containing at least one hydrophobic part having from 7 to 30 carbon atoms are chosen from acrylates and acrylamides of the following formula (1): 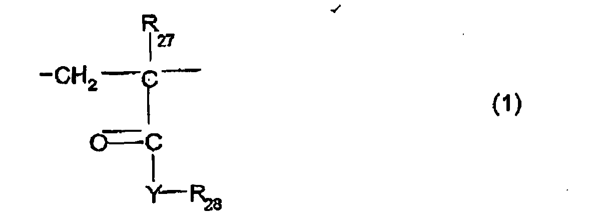 in which R₂₇ denotes a hydrogen atom, a linear or branched C₁-C₆ alkyl radical (preferably methyl); Y denotes O or NH; R₂₈ denotes a hydrophobic radical containing a fatty chain having from 7 to 22 carbon atoms, and preferably 7 to 18, and more particularly from 12 to 18 carbon atoms.

21. Composition according to Claim 20, where the hydrophobic radical R₂₈ is chosen from saturated or unsaturated, linear or branched C₇-C₁₈ alkyl radicals; C₇-C₁₈ perfluorinated alkyl radicals; the cholesteryl radical or a cholesterol ester; aromatic polycyclic groups.

22. Composition according to Claim 20 or 21, where the hydrophobic radical R₂₈ further contains at least one alkylene oxide unit and preferably one polyoxyalkylenated chain.

23. Composition according to Claim 22, where the number of moles of oxyalkylenated units varies from 1 to 30 mol and more preferably from 1 to 25 mol and more preferably still from 3 to 20 mol.

24. Composition according to any one of Claims 20 to 23, where the amphiphilic AMPS polymers are amphiphilic copolymers consisting:
(a) of 2-acrylamido-2-methylpropanesulphonic acid (AMPS) units of the following formula (2): 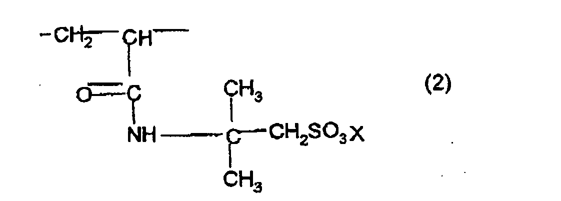 in which X⁺ is a proton, an alkali metal cation, an alkaline-earth metal cation or the ammonium ion;
(b) and of units of the following formula (3): 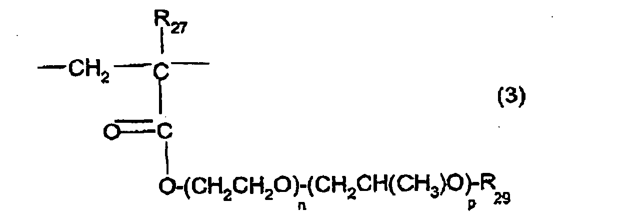
in which n and p, independently of each other, denote a number of moles and varies from 0 to 30, preferably from 1 to 25 and more preferably from 3 to 20, provided that n + p is less than or equal to 30, preferably less than 25 and better still less than 20; R₂₇ has the same meaning indicated above in formula (1) and R₂₉ denotes a linear or branched alkyl containing m carbon atoms ranging from 7 to 22, preferably from 7 to 18 carbon atoms and better still from 12 to 18 carbon atoms.

25. Composition according to Claim 24, where X⁺ denotes sodium or ammonium.

26. Composition according to Claim 24 or 25, where the amphiphilic AMPS polymers are chosen from:
(i) those which are non-crosslinked, for which p=0, n=7 or 25, R₂₇ denotes methyl and R₂₉ represents a mixture of C₁₂-C₁₄ or C₁₆-C₁₈ alkyl,
(ii) those which are crosslinked, for which p=0, n=8 or 25, R₂₇ denotes methyl and R₂₉ represents a mixture of C₁₆-C₁₈ alkyl.

27. Composition according to any one of Claims 24 to 26, where the molar proportion of units of formula (3) varies from 0.1 to 50%, more particularly from 1 to 25% and more particularly still from 3 to 10%.

28. Composition according to any one of Claims 24 to 26, where the molar proportion of units of formula (3) varies from 50.1 to 99.9% and more particularly from 60 to 95% and more particularly still from 65 to 90%.

29. Composition according to any one of Claims 1 to 28, where the AMPS polymers are present in quantities as active material ranging from 0.01 to 20% by weight, more preferably from 0.1 to 10% by weight, more preferably still from 0.1 to 5% by weight and more particularly still from 0.5 to 2% by weight relative to the total weight of the composition.

30. Composition according to any one of Claims 1 to 29, where the UV-A-screening agent of the 4,4-diarylbutadiene type corresponds to the following formula (I) : 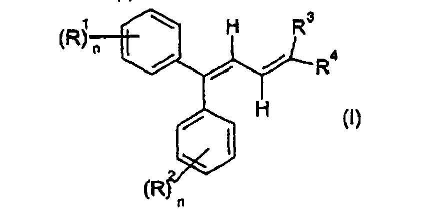 in which the diene system is of the Z,Z; Z,E; E,Z or E,E configuration or mixtures of the said configurations, and where:
- R¹ and R², which are identical or different, denote hydrogen, a C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a C₁-C₂₀ alkoxycarbonyl radical; a C₁-C₁₂ monoalkylamino radical; a C₁-C₁₂ dialkylamino radical; an aryl; a heteroaryl or a water-solubilizing substituent chosen from a carboxylate residue, a sulphonate residue or an ammonium residue;
- R³ denotes a group COOR⁵; COR⁵; CONR⁵R⁶; CN; O=S(-R⁵)=O; O=S(-OR⁵)=O; R⁷O-P-(-OR⁸)=O; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an optionally substituted C₆-C₁₈ aryl; an optionally substituted C₃-C₇ heteroaryl;
- R⁴ denotes a group COOR⁶; COR⁶; CONR⁵R⁶; CN; O=S(-R⁶)=O; O=S(-OR⁶)=O; R⁷O-P-(-OR⁸)=O; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an optionally substituted C₆-C₁₈ aryl; an optionally substituted C₃-C₇ heteroaryl;
- the radicals R⁵ to R⁸, which are identical or different, denote hydrogen; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ bicycloalkenyl radical; a C₇-C₁₀ cycloalkenyl radical; an optionally substituted aryl; an optionally substituted heteroaryl;
- n varies from 1 to 3;
the radicals R³ to R⁸ can form between them, with the carbon atoms to which they are attached, a C₅-C₆ ring which may be fused.

31. Composition according to Claim 30, where the compound of formula (I) is chosen from those for which
- n = 1 or 2;
- R¹ and R², which are identical or different, denote hydrogen, a C₁-C₂₀ alkyl radical; a C₁-C₁₂ alkoxy radical; a C₁-C₁₂ monoalkylamino radical; a C₁-C₁₂ dialkylamino radical; a water-solubilizing substituent chosen from a carboxylate group, a sulphonate group or an ammonium residue;
- R³ denotes a group COOR⁵; COR⁵; CONR⁵R⁶; a C₁-C₂₀ alkyl radical; a C₃-C₁₀ cycloalkyl radical; a C₃-C₁₀ cycloalkenyl radical; a C₇-C₁₀ bicycloalkyl radical; optionally substituted phenyl, naphthyl or thienyl;
- R⁴ denotes a group COOR⁶; COR⁶; CONR⁵R⁶; a C₁-C₂₀ alkyl radical; a C₃-C₆ cycloalkyl radical; a C₃-C₁₀ cycloalkenyl radical; a C₇-C₁₀ bicycloalkyl radical; optionally substituted phenyl, naphthyl or thienyl;
- the radicals R⁵ and R⁶, which are identical or different, denote hydrogen; a C₁-C₁₂ alkyl radical; a C₃-C₁₀ cycloalkyl radical; a C₃-C₁₀ cycloalkenyl radical; a C₇-C₁₀ bicycloalkyl radical; a C₃-C₁₀ bicycloalkenyl radical; optionally substituted phenyl or naphthyl.

32. Composition according to Claim 31, where the compound of formula (I) is chosen from those for which
- R¹ and R², which are identical or different, denote hydrogen, a C₁-C₂₀ alkyl radical; a C₁-C₂₀ alkoxy radical; a water-solubilizing substituent chosen from a carboxylate group, a sulphonate group or an ammonium residue;
- R³ denotes a group COOR⁵; COR⁵; CONR⁵R⁶;
- R⁴ denotes a group COOR⁶; COR⁶; CONR⁵R⁶;
- the radicals R⁵ and R⁶, which are identical or different, denote hydrogen; a C₁-C₁₂ alkyl radical; a C₃-C₆ cycloalkyl radical; a C₃-C₁₀ cycloalkenyl radical; a C₇-C₁₀ bicycloalkyl radical; a C₃-C₁₀ bicycloalkenyl radical; optionally substituted phenyl or naphthyl.

33. Composition according to Claim 32, where the compound of formula (I) is chosen from those of the following formula (I'): 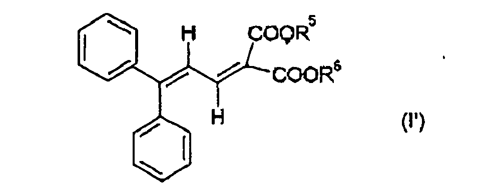 where the radicals R⁵ and R⁶, which are identical or different, denote hydrogen; a C₁-C₂₀ alkyl radical; a C₃-C₆ cycloalkyl radical; a C₃-C₁₀ cycloalkenyl radical.

34. Composition according to Claim 33, where the compound of formula (I') is 1,1-dicarboxy-(2'2'-dimethylpropyl)-4,4-diphenylbutadiene having the structure:

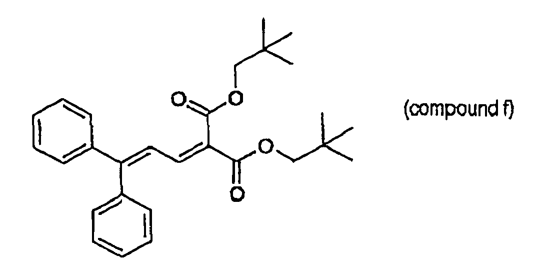

35. Composition according to any one of Claims 1 to 29, where the UV-A-screening agent of the 4,4-diarylbutadiene type corresponds to the following formula (II): 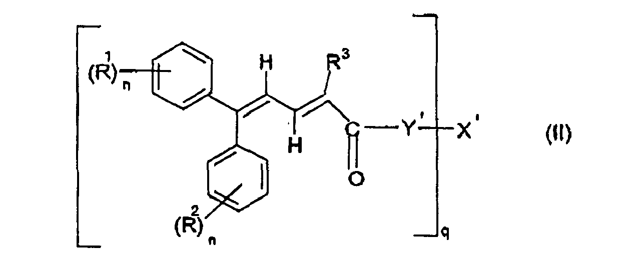 in which the diene system is of the Z,Z; Z,E; E,Z or E,E configuration or mixtures of the said configurations and where:
- R¹, R², R³ and n have the meanings indicated in the formula (I) as defined in Claim 30;
- Y' denotes a group -O- or -NR⁹-
- R⁹ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- X' denotes a residue of a linear or branched, aliphatic or cycloaliphatic C₂-C₂₀ polyol comprising from 2 to 10 hydroxyl groups and having the valency q; it being possible for the carbon chain of the said residue to be interrupted by one or more sulphur or oxygen atoms; one or more imine groups; one or more C₁-C₄ alkylimino groups;
- q ranges from 2 to 10.

36. Composition according to Claim 35, where the compound of formula (II) is chosen from those for which:
- R¹ and R², which are identical or different, denote hydrogen, a C₁-C₁₂ alkyl radical; a C₁-C₈ alkoxy radical; a water-solubilizing substituent chosen from a carboxylate group, a sulphonate group or an ammonium residue;
- R³ denotes a group COOR⁵; CONR⁵R⁶; CN; a C₃-C₁₀ cycloalkyl radical; a C₇-C₁₀ bicycloalkyl radical;
- R⁵ and R⁶, which are identical or different, denote a linear or branched C₁-C₂₀ alkyl radical; a C₃-C₁₀ cycloalkyl radical; a C₇-C₁₀ bicycloalkyl radical; optionally substituted naphthyl or phenyl;
- X' denotes a C₂-C₂₀ polyol residue comprising from 2 to 6 hydroxyl groups and more particularly from 2 to 4.

37. Composition according to Claim 36, where the compound of formula (II) is chosen from those for which X' denotes an ethanol or pentaerythritol residue.

38. Composition according to Claim 37, where the compound of formula (II) is chosen from the following compounds:

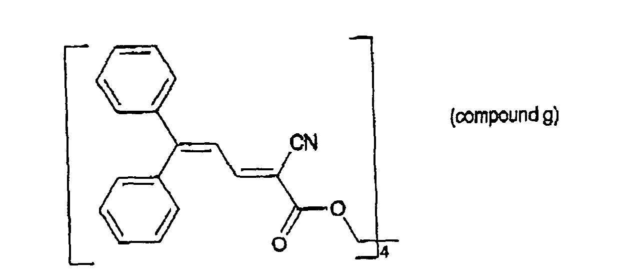

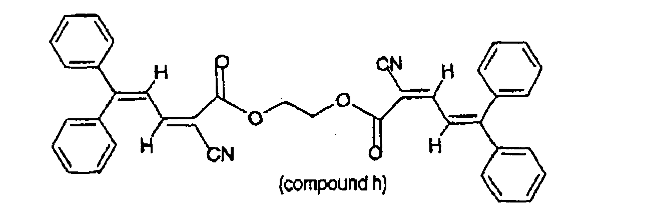

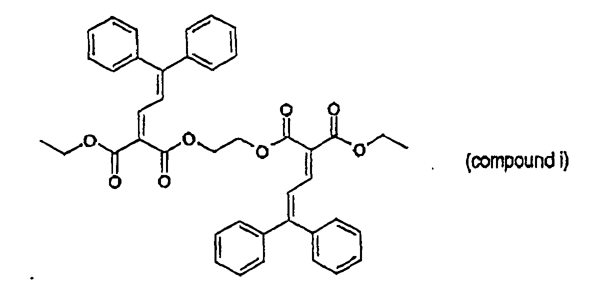

39. Composition according to any one of Claims 1 to 38, **characterized in that** the 4,4-diarylbutadiene compound(s) is/are present in proportions ranging from 0.1% to 20% by weight, more preferably from 1 to 10% by weight relative to the total weight of the emulsion.

40. Composition according to any one of Claims 1 to 39, **characterized in that** it additionally contains at least one additional organic or inorganic sunscreening agent active in UV-A and/or UV-B, water-soluble, fat-soluble or insoluble in the commonly used cosmetic solvents.

41. Composition according to Claim 40, where the additional organic screening agents are chosen from anthranilates; cinnamic derivatives; dibenzoylmethane derivatives; salicylic derivatives, camphor derivatives; triazine derivatives; benzophenone derivatives; β,β'-diphenyl acrylate derivatives; benzotriazole derivatives; benzalmalonate derivatives; benzimidazole derivatives; imidazolines; bis-benzoazolyl derivatives; p-aminobenzoic acid (PABA) derivatives; benzoxazole derivatives; methylenebis(hydroxyphenylbenzotriazole) derivatives; screening polymers and screening silicones; dimers derived from α-alkylstyrene and mixtures thereof.

42. Composition according to Claim 41, where the additional organic screening agents are chosen from
Ethylhexyl Salicylate,
Ethylhexyl Methoxycinnamate,
Octocrylene,
Butyl Methoxydibenzoylmethane,
Phenylbenzimidazole Sulphonic Acid,
Benzophenone-3,
Benzophenone-4,
Benzophenone-5,
n-Hexyl 2-(4-diethylamino-2-hydroxybenzoyl)benzoate,
4-Methylbenzylidene camphor,
Terephthalylidene Dicamphor Sulphonic Acid,
Disodium Phenyl Dibenzimidazole Tetra-sulphonate,
2,4,6-Tris(4'-diisobutyl aminobenzalmalonate)-s-triazine
Anisotriazine,
Ethylhexyl triazone,
Diethylhexyl Butamido Triazone,
Methylene bis-Berizotriazolyl Tetramethylbutylphenol,
Drometrizole Trisiloxane,
Polysilicone 15,
2,4-Bis-[5-1-(dimethylpropyl)benzoxazol-2-yl-(4-phenyl)imino]-6-(2-ethylhexyl)imino-1,3,5-triazine
and mixtures thereof.

43. Composition according to Claim 40, where the additional inorganic screening agents are chosen from pigments or nanopigments of metal oxides, coated or uncoated.

44. Composition according to Claim 43, where the additional inorganic screening agents are nanopigments of titanium oxide, which is amorphous or crystallized, in rutile and/or anatase form, iron oxide, zinc oxide, zirconium oxide or cerium oxide.

45. Composition according to any one of Claims 1 to 44, **characterized in that** it additionally contains at least one agent for artificial bronzing and/or tanning of the skin.

46. Composition according to any one of Claims 1 to 45, **characterized in that** it additionally contains at least one cosmetic adjuvant chosen from organic solvents, ionic or nonionic thickeners, demulcents, humectants, opacifying agents, stabilizers, emollients, silicones, insect repellents, perfumes, preservatives, surfactants, fillers, active agents, pigments, polymers, propellants, alkalizing or acidifying agents or any other ingredient customarily used in the cosmetic and/or dermatological field.

47. Composition according to any one of Claims 1 to 46, **characterized in that** it is provided in the form of an oil-in-water emulsion containing at most 1% by weight of emulsifying surfactant relative to the total weight of the composition.

48. Use of a composition as defined in any one of Claims 1 to 47, for the manufacture of products for the cosmetic treatment of the skin, the lips and the hair, including the scalp, in particular for the protection and/or care of the skin, the lips and/or the hair, and/or for making up the skin and/or the lips.

## Patentansprüche

1. Lichtschutzzusammensetzung, die mindestens eine wässrige Phase, mindestens eine Ölphase, mindestens ein wasserlösliches oder in Wasser dispergierbares Polymer der Acrylamido-2-methylpropansulfonsäure (AMPS), das ganz oder teilweise neutralisiert, vernetzt oder unvernetzt ist, und mindestens ein Filtersystem für UV-Strahlung enthält, **dadurch gekennzeichnet, dass** das Filtersystem mindestens ein UV-A-Filter vom Typ 4,4-Diarylbutadien umfasst; wobei die Zusammensetzung in Form einer Öl-in-Wasser-Emulsion vorliegt.

2. Zusammensetzung nach Anspruch 1, **dadurch gekennzeichnet, dass** das AMPS-Polymer ganz oder teilweise mit einer anorganischen oder organischen Base neutralisiert ist.

3. Zusammensetzung nach Anspruch 2, **dadurch gekennzeichnet, dass** die anorganische Base unter Natriumhydroxid, Kaliumhydroxid oder Ammoniak ausgewählt ist.

4. Zusammensetzung nach Anspruch 2, **dadurch gekennzeichnet, dass** die organische Phase unter Mono-, Di- oder Triethanolamin, Aminomethylpropandiol, N-Methylglucamin, basischen Aminosäuren und ihren Gemischen ausgewählt ist.

5. Zusammensetzung nach einem der Ansprüche 1 bis 4, in der das AMPS-Polymer zu mindestens 90 % neutralisiert ist.

6. Zusammensetzung nach einem der Ansprüche 1 bis 5, in der das AMPS-Polymer vernetzt ist und in der das Vernetzungsmittel unter den Verbindungen mit mehreren olefinisch ungesättigten Bindungen ausgewählt ist, die häufig zur Vernetzung der durch radikalische Polymerisation erhaltenen Polymere verwendet werden.

7. Zusammensetzung nach Anspruch 6, in der das Vernetzungsmittel ausgewählt ist unter Divinylbenzol, Diallylether, Dipropylenglycoldiallylether, Polyglycoldiallylethern, Triethylenglycoldivinylether, Hydrochinondiallylether, Ethylenglycoldi(meth)acrylat oder Tetraethylenglycoldi(meth)acrylat, Trimethylolpropantriacrylat, Methylen-bis-acrylamid, Methylen-bis-methacrylamid, Triallylamin, Triallylcyanurat, Diallylmaleat, Tetraallylethylendiamin, Tetraallyloxyethan, Trimethylolpropandiallylether, Allyl-(meth)acrylat, Allylethern von Alkoholen der Zuckergruppe, oder anderen Allyl- oder Vinylethern von polyfunktionellen Alkoholen, sowie Allylestern der Derivate der Phosphorsäure und/oder der Vinylphosphonsäure, oder ihren Gemischen.

8. Zusammensetzung nach Anspruch 6, in der das Vernetzungsmittel unter Methylen-bis-acrylamid, Allylmethacrylat oder Trimethylolpropantriacrylat (TMPTA) ausgewählt ist.

9. Zusammensetzung nach einem der Ansprüche 1 bis 8, in der der Vernetzungsgrad im Allgemeinen im Bereich von 0,01 bis 10 Mol-% und insbesondere von 0,2 bis 2 Mol-% liegt, bezogen auf das Polymer.

10. Zusammensetzung nach einem der Ansprüche 1 bis 9, **dadurch gekennzeichnet, dass** das AMPS-Polymer wasserlöslich oder in Wasser dispergierbar ist und ausgewählt ist unter:
(i) vernetzten oder nicht vernetzten Homopolymeren von AMPS;
(ii) vernetzten oder nicht vernetzten Copolymeren, die aus AMPS und einem oder mehreren hydrophilen Monomeren mit ethylenisch ungesättigter Bindung oder hydrophoben Monomeren mit ethylenisch ungesättigter Bindung, die keine Fettkette enthalten, erhalten werden.

11. Zusammensetzung nach Anspruch 10, in der die wasserlöslichen oder in Wasser dispergierbaren Polymere von AMPS eine Molmasse von 50 000 bis 10 000 000 g/mol, vorzugsweise von 80 000 bis 8 000 000 g/mol, und noch bevorzugter von 100 000 bis 7 000 000 g/mol aufweisen.

12. Zusammensetzung nach Anspruch 10 oder 11, in der das Homopolymer von AMPS das Ammoniumpolyacryloyldimethyltauramid ist.

13. Zusammensetzung nach Anspruch 10 oder 11, in der die vernetzten oder nicht vernetzten Copolymere aus AMPS und einem oder mehreren hydrophilen oder hydrophoben Monomeren mit ethylenisch ungesättigter Bindung ausgewählt sind unter
(a) den Copolymeren aus AMPS und Acrylamid oder Methylacrylamid;
(b) den Copolymeren aus AMPS und Vinylpyrrolidon oder Vinylformamid.

14. Zusammensetzung nach Anspruch 13, in der das wasserlösliche oder in Wasser dispergierbare Copolymer von AMPS ausgewählt ist unter:
- Polyacrylamid/C₁₃₋₁₄-Isoparaffin/Laureth-7
- Acrylamid/Natrium Acryloyldimethyltaurat/Isohexadecan/Polysorbat-80;
- Ammoniumacryloyldimethyltaurat/VP-Copolymer.

15. Zusammensetzung nach einem der Ansprüche 1 bis 9, **dadurch gekennzeichnet, dass** das AMPS-Polymer amphiphil ist.

16. Zusammensetzung nach Anspruch 15, in der das amphiphile Polymer von AMPS mindestens eine Fettkette umfasst, die 7 bis 30 Kohlenstoffatome, vorzugsweise 7 bis 22 Kohlenstoffatome und noch bevorzugter 7 bis 18 Kohlenstoffatome und insbesondere 12 bis 18 Kohlenstoffatome enthält.

17. Zusammensetzung nach Anspruch 15 oder 16, in der das amphiphile Polymer von AMPS ein Molekulargewicht von 50 000 bis 10 000 000, bevorzugter von 100 000 bis 8 000 000 und noch bevorzugter von 100 000 bis 7 000 000 aufweist.

18. Zusammensetzung nach einem der Ansprüche 15 bis 17, in der die amphiphilen Polymere von AMPS ausgewählt sind unter den statistischen amphiphilen Polymeren von AMPS, die durch Reaktion mit einem C₆₋₂₂-n-Monoalkylamin oder C₆₋₂₂-Di-n-alkylamin modifiziert sind und ein oder mehrere hydrophile Monomere mit ethylenisch ungesättigter Bindung enthalten können.

19. Zusammensetzung nach einem der Ansprüche 15 bis 17, in der die amphiphilen Polymere von AMPS ausgewählt sind unter den Polymeren von AMPS und mindestens einem Monomer mit ethylenisch ungesättigter Bindung, das mindestens einen hydrophoben Teil mit 7 bis 30 Kohlenstoffatomen und bevorzugter 7 bis 22 Kohlenstoffatomen und noch bevorzugter 7 bis 18 Kohlenstoffatomen und insbesondere 12 bis 18 Kohlenstoffatomen enthält, und gegebenenfalls einem oder mehreren hydrophilen Comonomeren mit ethylenisch ungesättigter Bindung.

20. Zusammensetzung nach Anspruch 19, in der die Monomere mit ethylenisch ungesättigter Bindung, die mindestens einen hydrophoben Teil mit 7 bis 30 Kohlenstoffatomen umfassen, unter den Acrylaten oder Acrylamiden der folgenden Formel (1) ausgewählt sind: 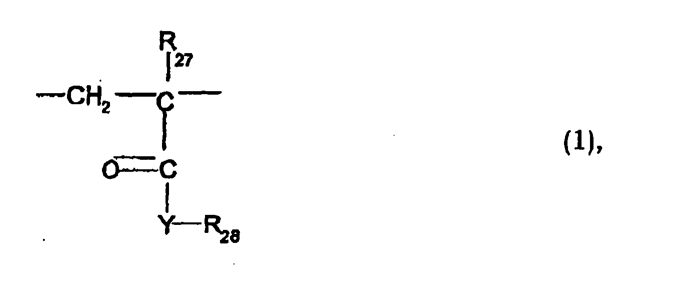 worin R₂₇ Wasserstoff, eine geradkettige oder verzweigte C₁₋₆-Alkylgruppe (vorzugsweise Methyl) bedeutet; Y O oder NH ist; R₂₈ eine hydrophobe Gruppe bedeutet, die eine Fettkette mit 7 bis 22 Kohlenstoffatomen, vorzugsweise 7 bis 18 und insbesondere 12 bis 18 Kohlenstoffatomen umfasst.

21. Zusammensetzung nach Anspruch 20, in der die hydrophobe Gruppe R₂₈ unter den geradkettigen oder verzweigten, gesättigten oder ungesättigten C₇₋₁₈-Alkylgruppen; den C₇₋₁₈-alkylperfluorierten Gruppen; der Cholesterylgruppe oder einem Cholesterinester; oder aromatischen polycyclischen Gruppen ausgewählt ist.

22. Zusammensetzung nach Anspruch 20 oder 21, in der die hydrophobe Gruppe R₂₈ darüber hinaus mindestens eine Alkylenoxideinheit und vorzugsweise eine polyalkoxylierte Kette umfasst.

23. Zusammensetzung nach Anspruch 22, in der die Molzahl der alkoxylierten Einheiten im Bereich von 1 bis 30 mol und vorzugsweise 1 bis 25 mol und noch bevorzugter 3 bis 20 mol liegt.

24. Zusammensetzung nach einem der Ansprüche 20 bis 23, in der die amphiphilen Polymere von AMPS amphiphile Copolymere sind, bestehend aus:
(a) Einheiten der 2-Acrylamido-2-methylpropansulfonsäure (AMPS) der folgenden Formel (2): 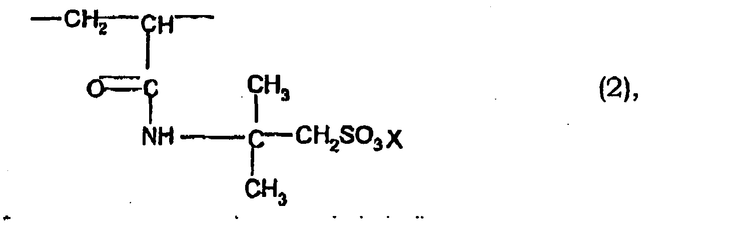 in der X⁺ ein Proton, ein Alkalimetallkation, ein Erdalkalimetallkation oder ein Ammoniumion ist;
(b) Einheiten der folgenden Formel (3): 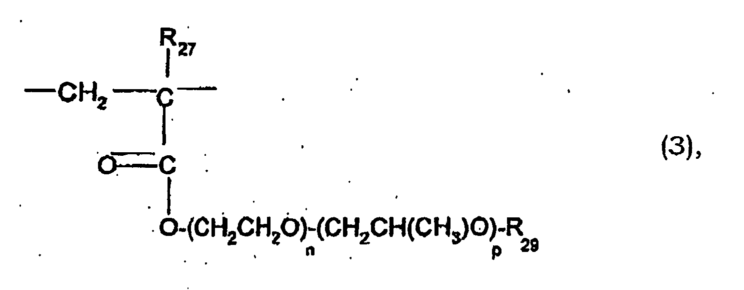 in der n und p jeweils unabhängig voneinander die Molzahl angeben und im Bereich von 0 bis 30, vorzugsweise von 1 bis 25 und noch bevorzugter von 3 bis 20 liegen, mit der Maßgabe, dass n + p kleiner oder gleich 30, vorzugsweise kleiner als 25 und noch besser kleiner als 20 ist; R₂₇ die oben in der Formel (1) angegebenen Bedeutungen aufweist; und R₂₉ eine geradkettige oder verzweigte Alkylgruppe bedeutet, die 7 bis 22 Kohlenstoffatome, vorzugsweise 7 bis 18 Kohlenstoffatome und noch besser 12 bis 18 Kohlenstoffatome umfasst.

25. Zusammensetzung nach Anspruch 24, in der X⁺ Natrium oder Ammonium ist.

26. Zusammensetzung nach Anspruch 24 oder 25, in der die amphiphilen Polymere von AMPS ausgewählt sind unter:
(i) den nicht vernetzten Polymeren, in denen p = 0, n = 7 oder 25, R₂₇ Methyl und R₂₉ ein Gemisch aus C₁₂₋₁₄-Alkyl oder C₁₆₋₁₈-Alkyl bedeutet,
(ii) den vernetzten Polymeren, in denen p = 0, n = 8 oder 25, R₂₇ Methyl und R₂₉ ein Gemisch aus C₁₆₋₁₈-Alkyl bedeutet.

27. Zusammensetzung nach einem der Ansprüche 24 bis 26, in der der molare Anteil der Einheiten der Formel (3) im Bereich von 0,1 bis 50 %, genauer von 1 bis 25 % und insbesondere von 3 bis 10 % liegt.

28. Zusammensetzung nach einem der Ansprüche 24 bis 26, in dem der molare Anteil der Einheiten der Formel (3) im Bereich von 50,1 bis 99,9 % und besonders von 60 bis 95 % und noch bevorzugter von 65 bis 90 % liegt.

29. Zusammensetzung nach einem der Ansprüche 1 bis 28, in der die Polymere von AMPS in wirksamen Mengen von 0,01 bis 20 Gew.-%, bevorzugter von 0,1 bis 10 Gew.-%, noch bevorzugter von 0,1 bis 5 Gew.-% und besonders bevorzugt von 0,5 bis 2 Gew.-%, bezogen auf das Gesamtgewicht der Zusammensetzung, enthalten sind.

30. Zusammensetzung nach einem der Ansprüche 1 bis 29, in der das UV-A-Filter vom Typ 4,4-Diarylbutadien der folgenden Formel (I) entspricht: 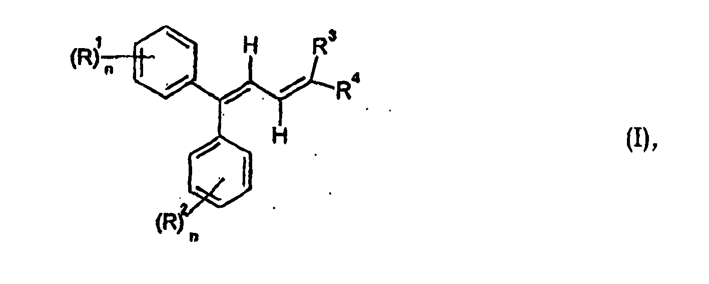 in der das Dien-System die Konfiguration Z,Z; Z,E; E,Z oder E,E oder Mischungen dieser Konfigurationen aufweist und in der bedeuten:
- R¹ und R², die gleich oder verschieden sind, Wasserstoff, eine C₁₋₂₀-Alkylgruppe, eine C₂₋₁₀-Alkenylgruppe, eine C₁₋₁₂-Alkoxygruppe, eine C₃₋₁₀-Cycloalkylgruppe, eine C₃₋₁₀-Cycloalkenylgruppe, eine C₁₋₂₀-Alkoxycarbonylgruppe, eine C₁₋₁₂-Monoalkylaminogruppe, eine C₁₋₁₂-Dialkylaminogruppe, Aryl, Heteroaryl oder einen in Wasser solubilisierenden Substituenten, der unter Carboxylat, Sulfonat oder Ammonium ausgewählt ist;
- R³ eine Gruppe COOR⁵, COR⁵, CONR⁵R⁶, CN, O=S(-R⁵)=O, O=S(-OR⁵)=O, R⁷O-P-(-OR⁸)=O, eine C₁₋₂₀-Alkylgruppe, eine C₂₋₁₀-Alkenylgruppe, eine C₃₋₁₀-Cycloalkylgruppe, eine C₇₋₁₀-Bicycloalkylgruppe, eine C₃₋₁₀-Cycloalkenylgruppe, eine C₇₋₁₀-Bicycloalkenylgruppe, eine C₆₋₁₈-Arylgruppe, die gegebenenfalls substituiert ist; eine C₃₋₇-Heteroarylgruppe, die gegebenenfalls substituiert ist;
- R⁴ eine Gruppe COOR⁶, COR⁶, CONR⁵R⁶, CN, O=S(-R⁶)=O, O=S(-OR⁶)=O, R⁷O-P-(-OR⁸)=O, eine C₁₋₂₀-Alkylgruppe, eine C₂₋₁₀-Alkenylgruppe, eine C₃₋₁₀-Cycloalkylgruppe, eine C₇₋₁₀-Bicycloalkylgruppe, eine C₃₋₁₀-Cycloalkenylgruppe, eine C₇₋₁₀-Bicycloalkenylgruppe, eine C₆₋₁₈-Azylgruppe, die gegebenenfalls substituiert ist, eine C₃₋₇-Heteroarylgruppe, die gegebenenfalls substituiert ist;
- R⁵ bis R⁸, die gleich oder verschieden sind, Wasserstoff; eine C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Bicycloalkenylgruppe; eine C₇₋₁₀-Cycloalkenylgruppe; eine Arylgruppe, die gegebenenfalls substituiert ist; eine Heteroarylgruppe, die gegebenenfalls substituiert ist;
- n 1 bis 3;
wobei die Gruppen R³ bis R⁸ mit den Kohlenstoffatomen, an die sie verbunden sind, einen C₅₋₆-Ring bilden können, der kondensiert sein kann.

31. Zusammensetzung nach Anspruch 30, in der die Verbindung der Formel (I) unter denen ausgewählt ist, für die bedeuten:
- n = 1 oder 2;
- R¹ und R², die gleich oder verschieden sind, Wasserstoff, eine C₁₋₂₀-Alkylgruppe; eine C₁₋₁₂-Alkoxygruppe; eine C₁₋₁₂-Monoalkylaminogruppe, eine C₁₋₁₂-Dialkylaminogruppe; einen in Wasser solubilisierenden Substituenten, der unter Carboxylat, Sulfonat oder Ammonium ausgewählt ist;
- R³ eine Gruppe COOR⁵, COR⁵, CONR⁵R⁶, eine C₁₋₂₀-Alkylgruppe; eine C₃₋₁₀-Cycloalkylgruppe; eine C₃₋₁₀-Cycloalkenylgruppe; eine C₇₋₁₀-Bicycloalkylgruppe; Phenyl, Naphthyl oder Thienyl, die gegebenenfalls substituiert sind;
- R⁴ eine Gruppe COOR⁶; COR⁶; CONR⁵R⁶; eine C₁₋₂₀-Alkylgruppe; eine C₃₋₆-Cycloalkylgruppe; eine C₃₋₁₀-Cycloalkenylgruppe; eine C₇₋₁₀-Bicycloalkylgruppe; Phenyl, Naphthyl oder Thienyl, die gegebenenfalls substituiert sind;
- R⁵ und R⁶, die gleich oder verschieden sind, Wasserstoff; eine C₁₋₁₂-Alkylgruppe; eine C₃₋₁₀-Cycloalkylgruppe; eine C₃₋₁₀-Cycloalkenylgruppe; eine C₇₋₁₀-Bicycloalkylgruppe; eine C₃₋₁₀-Bicycloalkenylgruppe; Phenyl oder Naphthyl, die gegebenenfalls substituiert sind.

32. Zusammensetzung nach Anspruch 31, in der die Verbindung der Formel (I) unter den Verbindungen ausgewählt ist, für die bedeuten:
- R¹ und R², die gleich oder verschieden sind, Wasserstoff, eine C₁₋₂₀-Alkylgruppe; eine C₁₋₂₀-Alkoxygruppe; einen in Wasser solubilisierenden Substituenten, der unter Carboxylat, Sulfonat oder Ammonium ausgewählt ist;
- R³ eine Gruppe COOR⁵; COR⁵; CONR⁵R⁶;
- R⁴ eine Gruppe COOR⁶; COR⁶; CONR⁵R⁶;
- R⁵ und R⁶, die gleich oder verschieden sind, Wasserstoff; eine C₁₋₁₂-Alkylgruppe; eine C₃₋₆-Cycloalkylgruppe; eine C₃₋₁₀-Cycloalkenylgruppe; eine C₇₋₁₀-Bicycloalkylgruppe; eine C₃₋₁₀-Bicycloalkenylgruppe; Phenyl oder Naphthyl, die gegebenenfalls substituiert sind.

33. Zusammensetzung nach Anspruch 32, in der die Verbindung der Formel (I) unter denen der folgenden Formel (I') ausgewählt ist: 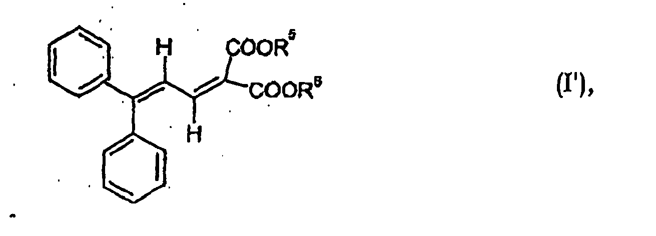 in denen R⁵ und R⁶, die gleich oder verschieden sind, Wasserstoff; eine C₁₋₂₀-Alkylgruppe; eine C₃₋₆-Cycloalkylgruppe; eine C₃₋₁₀-Cycloalklenylgruppe bedeuten.

34. Zusammensetzung nach Anspruch 33, in der die Verbindung der Formel (I') das 1,1-Dicarboxy-(2',2'-dimethyl-propyl)-4,4-diphenylbutadien der folgenden Struktur ist:

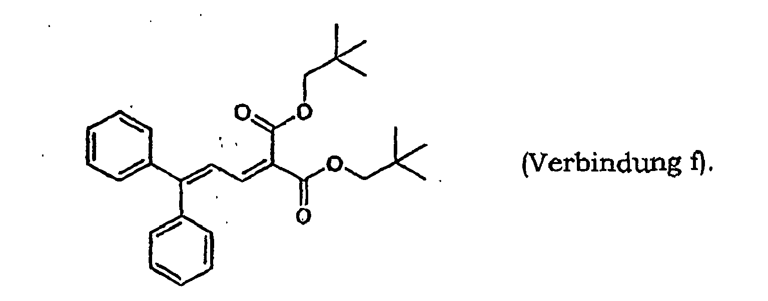

35. Zusammensetzung nach einem der Ansprüche 1 bis 29, in der das UV-A-Filter vom Typ 4,4-Diarylbutadien der folgenden Formel (II) entspricht: 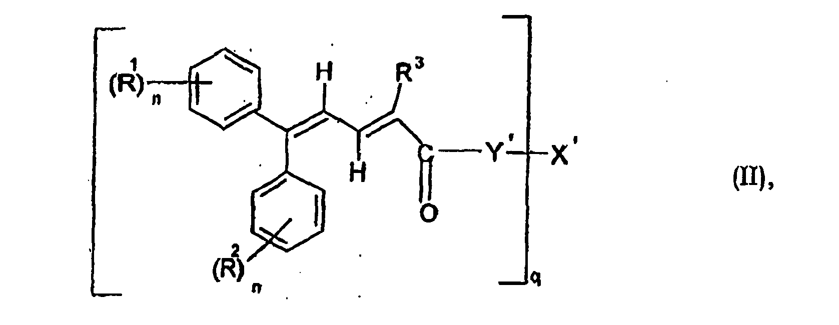 in der das Dien-System die Konfiguration Z,Z; Z,E; E,Z oder E,E oder Mischungen dieser Konfigurationen hat und worin bedeuten:
- R¹, R², R³ und n die in Anspruch 30 in der Formel (I) angegebenen Bedeutungen;
- Y' eine Gruppe -O- oder -NR⁹-;
- R⁹ Wasserstoff; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicyloalkylgruppe; eine C₃₋₁₀-Cycloalkenylgruppe; eine C₇₋₁₀-Bicycloalkenylgruppe; Aryl; Heteroaryl;
- X' einen geradkettigen oder verzweigten, aliphatischen oder cycloaliphatischen C₂₋₂₀-Polyolrest, die 2 bis 10 Hydroxygruppen und die Valenz q aufweist; wobei die Kohlenstoffkette dieses Restes unterbrochen sein kann durch ein oder mehrere Schwefel- oder Sauerstoffatome; eine oder mehrere Imingruppen; eine oder mehrere C₁₋₄-Alkyliminogruppen;
- q 2 bis 10.

36. Zusammensetzung nach Anspruch 35, in der die Verbindung der Formel (II) unter den Verbindungen ausgewählt ist, für die bedeuten:
- R¹ und R², die gleich oder verschieden sind, Wasserstoff, eine C₁₋₁₂-Alkylgruppe; eine C₁₋₈-Alkoxygruppe; einen in Wasser solubilisierenden Substituenten, der unter Carboxylat, Sulfonat oder Ammonium ausgewählt ist;
- R³ eine Gruppe COOR⁵; CONR⁵R⁶; CN; eine C₃₋₁₀-Cycloalkylgruppe; eine C₇₋₁₀-Bicycloalkylgruppe;
- R⁵ und R⁶, die gleich oder verschieden sind, eine geradkettige oder verzweigte C₁₋₂₀-Alkylgruppe; eine C₃₋₁₀-Cycloalkylgruppe; eine C₇₋₁₀-Bicycloalkylgruppe; Naphthyl oder Phenyl, die gegebenenfalls substituiert sind;
- X' einen C₂₋₂₀-Polyolrest, der 2 bis 6 Hydroxygruppen und insbesondere 2 bis 4 Hydroxygruppen enthält.

37. Zusammensetzung nach Anspruch 36, in der die Verbindung der Formel (II) unter denen ausgewählt ist, für die X' einen Ethanolrest oder Pentaerythritolrest bedeutet.

38. Zusammensetzung nach Anspruch 37, in der die Verbindung der Formel (II) unter den folgenden Verbindungen ausgewählt ist:

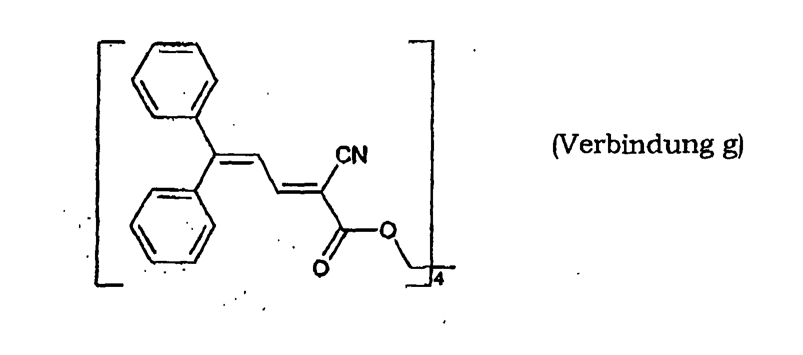

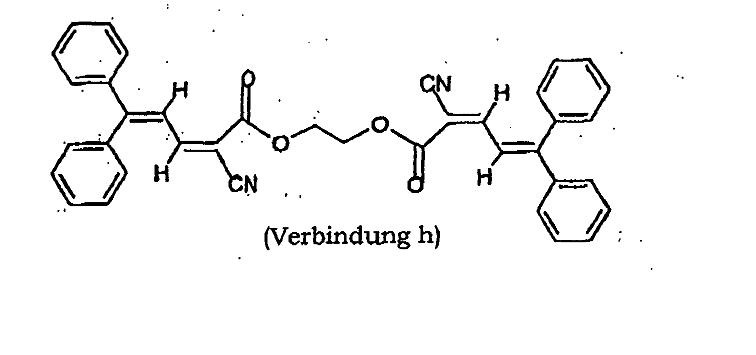

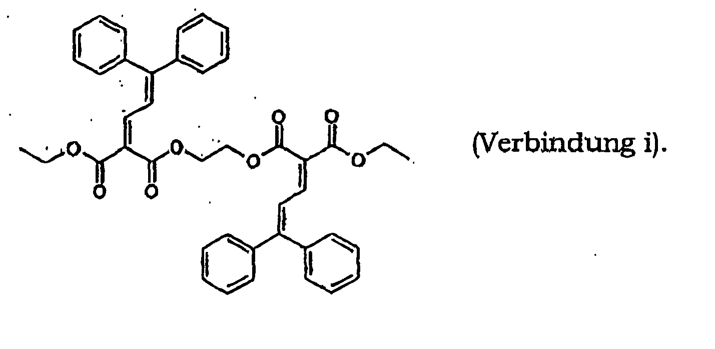

39. Zusammensetzung nach einem der Ansprüche 1 bis 38, **dadurch gekennzeichnet, dass** das oder die 4,4-Diarylbutadien(e) in einem Anteil von 0,1 bis 20 Gew.-%, und vorzugsweise 1 bis 10 Gew.-%, bezogen auf das Gesamtgewicht der Emulsion, vorliegen.

40. Zusammensetzung nach einem der Ansprüche 1 bis 39, **dadurch gekennzeichnet, dass** sie darüber hinaus mindestens ein zusätzliches, organisches oder anorganisches Sonnenschutzfilter umfasst, das im UV-A- und/oder UV-B-Bereich wirksam ist, sowie in den üblicherweise verwendeten kosmetischen Lösungsmitteln wasserlöslich, fettlöslich oder auch unlöslich ist.

41. Zusammensetzung nach Anspruch 40, in der die ergänzenden organischen Filter unter den Anthranilaten; Zimtsäurederivaten; Dibenzoylmethanderivaten; Salicylsäurederivaten, Campherderivaten; Triazinderivaten; Benzophenonderivaten; Derivaten von β,β-Diphenylacrylat; Benzotriazolderivaten; Benzalmalonatderivaten; Derivaten von Benzimidazol; Imidazolinen; Bis-benzoazolylderivaten; Derivaten von *p*-Aminobenzoesäure (PABA); Benzoxazolderivaten; Derivaten von Methylen-bis(hydroxyphenyl-benzotriazol); Polymerfiltern und Siliconfiltern; dimeren Derivaten von α-Alkylstyrol und ihren Gemischen ausgewählt sind.

42. Zusammensetzung nach Anspruch 41, in der die ergänzenden organischen Filter ausgewählt sind unter
Ethylhexyl Salicylate,
Ethylhexyl Methoxycinnamate,
Octocrylene,
Butyl Methoxydibenzoylmethane,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene Camphor,
Terephthalylidene Dicamphor Sulfonic Acid,
Disodium Phenyl Dibenzimidazole Tetrasulfonate,
2,4,6-Tris-(diisobutyl-4'amino-benzalmalonat)-s-triazin,
Anisotriazine,
Ethylhexyl Triazone,
Diethylhexyl Butamido Triazone,
Methylen bis-Benzotriazolyl Tetramethylbutylphenol,
Drometrizole Trisiloxane,
Polysilicone-15,
2,4-Bis[5-1-(dimethylpropyl)benzoxazol-2-yl-(4-phenyl)-imino]-6-(2-ethylhexyl)-imino-1,3,5-triazin und ihren Gemischen.

43. Zusammensetzung nach Anspruch 40, in der die anorganischen ergänzenden Filter unter den gegebenenfalls umhüllten Pigmenten oder Nanopigmenten von Metalloxiden ausgewählt sind.

44. Zusammensetzung nach Anspruch 43, in der die ergänzenden anorganischen Filter Nanopigmente von amorphem oder kristallinem Titanoxid, in Form von Rutil und/oder Anatas, Eisenoxid, Zinkoxid, Zirconiumoxid oder Ceroxid sind.

45. Zusammensetzung nach einem der Ansprüche 1 bis 44, **dadurch gekennzeichnet, dass** sie darüber hinaus mindestens ein Bräunungsmittel und/oder Mittel zur künstlichen Braunfärbung der Haut enthält.

46. Zusammensetzung nach einem der Ansprüche 1 bis 45, **dadurch gekennzeichnet, dass** sie darüber hinaus mindestens einen kosmetischen Zusatzstoff enthält, der unter den organischen Lösungsmitteln, ionischen oder nichtionischen Verdickungsmitteln, reizlindernden Stoffen, Feuchthaltemitteln, Trübungsmitteln, Stabilisatoren, Emollientien, Siliconen, insektensabwehrenden Wirkstoffen, Parfums, Konservierungsmitteln, grenzflächenaktiven Stoffen, Füllstoffen, Wirkstoffen, Pigmenten, Polymeren, Treibmitteln, Alkalisierungsmitteln oder Ansäuerungsmitteln oder beliebigen anderen Bestandteilen, die gewöhnlich auf dem Gebiet der Kosmetik und/oder Dermatologie verwendet werden, ausgewählt ist.

47. Zusammensetzung nach einem der Ansprüche 1 bis 46, **dadurch gekennzeichnet, dass** sie in Form einer Öl-in-Wasser-Emulsion vorliegt, die mehr als 1 Gew.-% eines emulgierenden Tensids, bezogen auf das Gesamtgewicht der Zusammensetzung, enthält.

48. Verwendung einer Zusammensetzung, wie sie in einem der Ansprüche 1 bis 47 definiert wurde, zur Herstellung eines Produkts zur kosmetischen Behandlung der Haut, der Lippen und der Haare, wobei die Kopfhaut eingeschlossen ist, insbesondere zum Schutz und/oder zur Pflege der Haut, der Lippen und/oder der Haare und/oder als Schminkprodukt für die Haut und/oder die Lippen.
